# EUROPEAN PATENT APPLICATION

(11) **EP 4 793 366 A1**
(43) Date of publication of application: **19.08.2026**
(21) Application number: 24877158.6
(22) Date of filing: 07.10.2024
(51) Int. Cl.: C12Q 1/6869, C12Q 1/686

(54) **METHOD FOR DEGRADING 5'-ADENYLATED SINGLE-STRANDED DNA, METHOD FOR LIGATING 3' ADAPTER TO NUCLEIC ACID, METHOD FOR PREPARING NGS LIBRARY, KIT FOR PREPARING NGS LIBRARY, AND 3' ADAPTER FOR PREPARING NUCLEIC ACID LIBRARY**

(30) Priority: 13.10.2023 JP 2023177841
(71) Applicant: ARKRAY, Inc., Kyoto-shi, Kyoto 601-8045 (JP)
(72) Inventor: NISHIDA, Ruisa, Kyoto-shi, Kyoto 602-0008 (JP); HOJO, Hiroaki, Kyoto-shi, Kyoto 602-0008 (JP); OTOMO, Ryo, Kyoto-shi, Kyoto 602-0008 (JP)
(74) Representative: MFG Patentanwälte Meyer-Wildhagen Meggle-Freund Gerhard PartG mbB
(86) International application number: PCT/JP2024/035863
(87) International publication number: WO 2025/079563

(57) **Abstract**

A method for degrading 5'-adenylated single-stranded DNA includes degrading the 5'-adenylated single-stranded DNA with RecJ. A base sequence at the 5'-end of the 5'-adenylated single-stranded DNA, exclusive of adenylic acid at the 5'-end, is a specific base sequence.

## Description

### Technical Field

The present disclosure relates to a method for degrading 5'-adenylated single-stranded DNA, a method for ligating 3' adapter to a nucleic acid, a method for preparing an NGS library, a kit for preparing an NGS library, and a 3' adapter for preparing a nucleic acid library.

### Background Art

In recent years, comprehensive quantitative analysis of nucleic acid, represented by Next-Generation Sequencing (also referred to as NGS, hereinafter), has been applied to discrimination of diseases and the like.

In preparation of a library for NGS, a ligation operation is conducted to add base sequences called 5' adapter and 3' adapter, respectively to the 5'-end and the 3'-end of a target base sequence to be sequenced. The ligation operation, however, also causes a side reaction of producing an adapter dimer, as a result of binding of excessive 5' adapter and excessive 3' adapter not ligated to the target base sequence. Polymerase chain reaction (also referred to as PCR, hereinafter) of the adapter dimer will therefore occur, concurrently with PCR of the target base sequence. That is, sequencing efficiency (or read counts per data volume) in the subsequent sequencing will decrease, since sequence reads ascribed to the adapter dimer will also be acquired. In one known method for suppressing the production of adapter dimer and improving sequencing efficiency, the ligation of 3' adapter to the target base sequence is followed by degradation of the excessive 3' adapter with a deadenylase called TAP, and with an exonuclease called RecJ, and then by ligation of the 5' adapter (see Patent Document 1, for example).

Patent Document 1: WO 2011/056866 A

### SUMMARY OF INVENTION

### Technical Problem

In the 3' adapter, the nucleotide at the 5'-end is adenylated, which is necessary for ligation to the 3'-end of the target base sequence. According to Patent Document 1, the adenylated 3' adapter cannot be degraded as it is by RecJ. The method therefore employs a technique by which the ligation of the 3' adapter is followed by deadenylation of the excessive 3' adapter, and further by degradation with RecJ. The method of Patent Document 1, however, requires multi-step enzyme treatment, and for which simplification of the procedure is desired.

Considering the circumstances, an object of the present disclosure is to provide a method for degrading 5'-adenylated single-stranded DNA, a method for ligating 3' adapter to a nucleic acid, a method for preparing an NGS library, a kit for preparing an NGS library, and a 3' adapter for preparing a nucleic acid library, for enabling rapid and easy preparation of an NGS library, with use of a single-stranded DNA having the nucleotide at the 5'-end that is adenylated, as the 3' adapter.

### Solution to Problem

Specific means for solving the aforementioned problems include the following aspects.
<1> A method for degrading 5'-adenylated single-stranded DNA, the method includes degrading the 5'-adenylated single-stranded DNA with RecJ, with a base sequence at the 5'-end of the 5'-adenylated single-stranded DNA, exclusive of adenylic acid at the 5'-end, being any one of the base sequences listed in the following Table A:

### [Table 1]

**Table A**

| No. | Base sequence (5' to 3') | No. | Base sequence (5' to 3') | No. | Base sequence (5' to 3') | No. | Base sequence (5' to 3') |
|---|---|---|---|---|---|---|---|
| 1 | GTTA | 46 | TTTC | 91 | TATG | 136 | CCTA |
| 2 | TAAT | 47 | ACAT | 92 | GAGA | 137 | GAGT |
| 3 | GTAT | 48 | GCTA | 93 | TGAT | 138 | AGTA |
| 4 | TGTT | 49 | TCTG | 94 | CTCA | 139 | CGAT |
| 5 | GTTT | 50 | ATTG | 95 | CATA | 140 | CCGT |
| 6 | TTAA | 51 | ATAA | 96 | CTAA | 141 | GGCA |
| 7 | GTTC | 52 | CGTT | 97 | GCAA | 142 | CATG |
| 8 | TACT | 53 | CTTC | 98 | TTGG | 143 | AAGT |
| 9 | CTTT | 54 | GTTG | 99 | CTAG | 144 | ACAA |
| 10 | TCAT | 55 | GTCG | 100 | TAAC | 145 | AGTC |
| 11 | TATA | 56 | CTTG | 101 | TAAA | 146 | CGTA |
| 12 | GATT | 57 | TGTA | 102 | ATCC | 147 | TCCA |
| 13 | TATC | 58 | ACTA | 103 | TCAG | 148 | CCCT |
| 14 | TTTG | 59 | GAAT | 104 | ACTG | 149 | TACC |
| 15 | TTCA | 60 | TCCT | 105 | ATAG | 150 | GGTC |
| 16 | TTAC | 61 | TTTT | 106 | CCTT | 151 | ACCA |
| 17 | TTCT | 62 | GACT | 107 | GAAA | 152 | CGTC |
| 18 | CATT | 63 | TAGT | 108 | CTGA | 153 | TAGG |
| 19 | TCTA | 64 | CAAT | 109 | CCAT | 154 | AAAA |
| 20 | TTGT | 65 | ATTC | 110 | GCGT | 155 | ACAG |
| 21 | TTCC | 66 | TCTC | 111 | TCAA | 156 | CACA |
| 22 | GTCA | 67 | AAAT | 112 | CATC | 157 | ACAC |
| 23 | CTAT | 68 | CTCT | 113 | TTGC | 158 | CGTG |
| 24 | AGTT | 69 | GTAG | 114 | CTAC | 159 | ATGC |
| 25 | ATTT | 70 | ATGT | 115 | ACGT | 160 | TCCG |
| 26 | TTAT | 71 | ATAC | 116 | TCGG | 161 | GCAG |
| 27 | GTCT | 72 | AATA | 117 | ACTC | 162 | ATGG |
| 28 | GTAA | 73 | ATGA | 118 | AACA | 163 | CAGA |
| 29 | ATAT | 74 | TTGA | 119 | TGTG | 164 | TAGA |
| 30 | GCTT | 75 | TCAC | 120 | CTGG | 165 | TAGC |
| 31 | CTTA | 76 | TCGT | 121 | TCGC | 166 | TGCT |
| 32 | GGTT | 77 | ACTT | 122 | ACGA | 167 | AACG |
| 33 | TCTT | 78 | GGTA | 123 | CAGT | 168 | CAAA |
| 34 | TTAG | 79 | AACT | 124 | CTCC | 169 | TGAG |
| 35 | ATCT | 80 | TACA | 125 | TCCC | 170 | ACGG |
| 36 | GATA | 81 | CTCG | 126 | TCGA | 171 | TGAA |
| 37 | TATT | 82 | GCTC | 127 | CTGC | 172 | AAGA |
| 38 | ATTA | 83 | AATC | 128 | GCTG | 173 | CACG |
| 39 | GTGA | 84 | GCAT | 129 | TGTC | 174 | GGAT |
| 40 | GACA | 85 | CACT | 130 | CCTG | 175 | TGGA |
| 41 | TTTA | 86 | GTAC | 131 | TACG | 176 | AGTG |
| 42 | AATT | 87 | GCGA | 132 | GTGG | 177 | CCAC |
| 43 | TTCG | 88 | ATCG | 133 | CCTC | 178 | GTCC |
| 44 | GATC | 89 | GTGT | 134 | ACCT | 179 | AATG |
| 45 | ATCA | 90 | CTGT | 135 | TAAG | 180 | GCGG |

<2> The method for degrading 5'-adenylated single-stranded DNA according to <1>, in which the base sequence at the 5'-end is any one selected from the group consisting of the base sequence Nos. 1 to 118 listed in the Table A.

<3> The method for degrading 5'-adenylated single-stranded DNA according to <1> or <2>, wherein, in a case in which any enzyme other than RecJ is also present, the enzyme other than RecJ allows the 5'-adenylated single-stranded DNA to remain adenylated, during the degrading.

<4> A method for ligating a 3' adapter to a nucleic acid, the method includes: ligating 5'-adenylated single-stranded DNA, as the 3' adapter, to the 3'-end of the nucleic acid; and after the ligating of the 5'-adenylated single-stranded DNA, degrading excessive 5'-adenylated single-stranded DNA by the method for degrading according to any one of <1> to <3>.

<5> A method for preparing an NGS library, the method includes: ligating 5'-adenylated single-stranded DNA, as a 3' adapter, to each 3'-end of a plurality of types of RNA having different sequences; after the ligating of the 5'-adenylated single-stranded DNA, degrading excessive 5'-adenylated single-stranded DNA by the method for degrading according to any one of <1> to <3>; ligating single-stranded RNA, as a 5' adapter, to each 5'-end of the plurality of types of RNA; synthesizing cDNA from the plurality of types of RNA ligated with the 3' adapter and the 5' adapter by a reverse transcription reaction; and amplifying the obtained cDNA by PCR.

<6> The method for preparing an NGS library according to <5>, wherein, in the degrading, in a case in which any enzyme other than RecJ is also present, the enzyme other than RecJ allows the 5'-adenylated single-stranded DNA to remain adenylated.

<7> The method for preparing an NGS library according to <5> or <6>, in which the plurality of types of RNA are small RNAs.

<8> A kit for preparing an NGS library, the kit includes: a 5'-adenylated single-stranded DNA stored in a first container; and RecJ stored in a second container, with a base sequence at the 5'-end of the 5'-adenylated single-stranded DNA, exclusive of adenylic acid at the 5'-end, being any one of the base sequences listed in the following Table A:

### [Table 2]

**Table A**

| No. | Base sequence (5' to 3') | No. | Base sequence (5' to 3') | No. | Base sequence (5' to 3') | No. | Base sequence (5' to 3') |
|---|---|---|---|---|---|---|---|
| 1 | GTTA | 46 | TTTC | 91 | TATG | 136 | CCTA |
| 2 | TAAT | 47 | ACAT | 92 | GAGA | 137 | GAGT |
| 3 | GTAT | 48 | GCTA | 93 | TGAT | 138 | AGTA |
| 4 | TGTT | 49 | TCTG | 94 | CTCA | 139 | CGAT |
| 5 | GTTT | 50 | ATTG | 95 | CATA | 140 | CCGT |
| 6 | TTAA | 51 | ATAA | 96 | CTAA | 141 | GGCA |
| 7 | GTTC | 52 | CGTT | 97 | GCAA | 142 | CATG |
| 8 | TACT | 53 | CTTC | 98 | TTGG | 143 | AAGT |
| 9 | CTTT | 54 | GTTG | 99 | CTAG | 144 | ACAA |
| 10 | TCAT | 55 | GTCG | 100 | TAAC | 145 | AGTC |
| 11 | TATA | 56 | CTTG | 101 | TAAA | 146 | CGTA |
| 12 | GATT | 57 | TGTA | 102 | ATCC | 147 | TCCA |
| 13 | TATC | 58 | ACTA | 103 | TCAG | 148 | CCCT |
| 14 | TTTG | 59 | GAAT | 104 | ACTG | 149 | TACC |
| 15 | TTCA | 60 | TCCT | 105 | ATAG | 150 | GGTC |
| 16 | TTAC | 61 | TTTT | 106 | CCTT | 151 | ACCA |
| 17 | TTCT | 62 | GACT | 107 | GAAA | 152 | CGTC |
| 18 | CATT | 63 | TAGT | 108 | CTGA | 153 | TAGG |
| 19 | TCTA | 64 | CAAT | 109 | CCAT | 154 | AAAA |
| 20 | TTGT | 65 | ATTC | 110 | GCGT | 155 | ACAG |
| 21 | TTCC | 66 | TCTC | 111 | TCAA | 156 | CACA |
| 22 | GTCA | 67 | AAAT | 112 | CATC | 157 | ACAC |
| 23 | CTAT | 68 | CTCT | 113 | TTGC | 158 | CGTG |
| 24 | AGTT | 69 | GTAG | 114 | CTAC | 159 | ATGC |
| 25 | ATTT | 70 | ATGT | 115 | ACGT | 160 | TCCG |
| 26 | TTAT | 71 | ATAC | 116 | TCGG | 161 | GCAG |
| 27 | GTCT | 72 | AATA | 117 | ACTC | 162 | ATGG |
| 28 | GTAA | 73 | ATGA | 118 | AACA | 163 | CAGA |
| 29 | ATAT | 74 | TTGA | 119 | TGTG | 164 | TAGA |
| 30 | GCTT | 75 | TCAC | 120 | CTGG | 165 | TAGC |
| 31 | CTTA | 76 | TCGT | 121 | TCGC | 166 | TGCT |
| 32 | GGTT | 77 | ACTT | 122 | ACGA | 167 | AACG |
| 33 | TCTT | 78 | GGTA | 123 | CAGT | 168 | CAAA |
| 34 | TTAG | 79 | AACT | 124 | CTCC | 169 | TGAG |
| 35 | ATCT | 80 | TACA | 125 | TCCC | 170 | ACGG |
| 36 | GATA | 81 | CTCG | 126 | TCGA | 171 | TGAA |
| 37 | TATT | 82 | GCTC | 127 | CTGC | 172 | AAGA |
| 38 | ATTA | 83 | AATC | 128 | GCTG | 173 | CACG |
| 39 | GTGA | 84 | GCAT | 129 | TGTC | 174 | GGAT |
| 40 | GACA | 85 | CACT | 130 | CCTG | 175 | TGGA |
| 41 | TTTA | 86 | GTAC | 131 | TACG | 176 | AGTG |
| 42 | AATT | 87 | GCGA | 132 | GTGG | 177 | CCAC |
| 43 | TTCG | 88 | ATCG | 133 | CCTC | 178 | GTCC |
| 44 | GATC | 89 | GTGT | 134 | ACCT | 179 | AATG |
| 45 | ATCA | 90 | CTGT | 135 | TAAG | 180 | GCGG |

<9> The kit for preparing an NGS library according to <8>, in which the nucleotide at the 3'-end of the 5'-adenylated single-stranded DNA is modified with a compound having a modification structure capable of suppressing binding of nucleic acid.

<10> The kit for preparing an NGS library according to <8> or <9>, further including at least one selected from the group consisting of 5' adapter, ligase, reverse transcriptase, reverse transcription primer, PCR enzyme, and PCR primer, in a manner such that each is stored in a separate container.

<11> A 3' adapter for preparing a nucleic acid library, the 3' adapter being a single-stranded DNA having the nucleotide at the 5'-end that is adenylated, and a base sequence at the 5'-end of the single-stranded DNA, exclusive of adenylic acid at the 5'-end, being any one of the base sequences listed in the following Table A:

### [Table 3]

**Table A**

| No. | Base sequence (5' to 3') | No. | Base sequence (5' to 3') | No. | Base sequence (5' to 3') | No. | Base sequence (5' to 3') |
|---|---|---|---|---|---|---|---|
| 1 | GTTA | 46 | TTTC | 91 | TATG | 136 | CCTA |
| 2 | TAAT | 47 | ACAT | 92 | GAGA | 137 | GAGT |
| 3 | GTAT | 48 | GCTA | 93 | TGAT | 138 | AGTA |
| 4 | TGTT | 49 | TCTG | 94 | CTCA | 139 | CGAT |
| 5 | GTTT | 50 | ATTG | 95 | CATA | 140 | CCGT |
| 6 | TTAA | 51 | ATAA | 96 | CTAA | 141 | GGCA |
| 7 | GTTC | 52 | CGTT | 97 | GCAA | 142 | CATG |
| 8 | TACT | 53 | CTTC | 98 | TTGG | 143 | AAGT |
| 9 | CTTT | 54 | GTTG | 99 | CTAG | 144 | ACAA |
| 10 | TCAT | 55 | GTCG | 100 | TAAC | 145 | AGTC |
| 11 | TATA | 56 | CTTG | 101 | TAAA | 146 | CGTA |
| 12 | GATT | 57 | TGTA | 102 | ATCC | 147 | TCCA |
| 13 | TATC | 58 | ACTA | 103 | TCAG | 148 | CCCT |
| 14 | TTTG | 59 | GAAT | 104 | ACTG | 149 | TACC |
| 15 | TTCA | 60 | TCCT | 105 | ATAG | 150 | GGTC |
| 16 | TTAC | 61 | TTTT | 106 | CCTT | 151 | ACCA |
| 17 | TTCT | 62 | GACT | 107 | GAAA | 152 | CGTC |
| 18 | CATT | 63 | TAGT | 108 | CTGA | 153 | TAGG |
| 19 | TCTA | 64 | CAAT | 109 | CCAT | 154 | AAAA |
| 20 | TTGT | 65 | ATTC | 110 | GCGT | 155 | ACAG |
| 21 | TTCC | 66 | TCTC | 111 | TCAA | 156 | CACA |
| 22 | GTCA | 67 | AAAT | 112 | CATC | 157 | ACAC |
| 23 | CTAT | 68 | CTCT | 113 | TTGC | 158 | CGTG |
| 24 | AGTT | 69 | GTAG | 114 | CTAC | 159 | ATGC |
| 25 | ATTT | 70 | ATGT | 115 | ACGT | 160 | TCCG |
| 26 | TTAT | 71 | ATAC | 116 | TCGG | 161 | GCAG |
| 27 | GTCT | 72 | AATA | 117 | ACTC | 162 | ATGG |
| 28 | GTAA | 73 | ATGA | 118 | AACA | 163 | CAGA |
| 29 | ATAT | 74 | TTGA | 119 | TGTG | 164 | TAGA |
| 30 | GCTT | 75 | TCAC | 120 | CTGG | 165 | TAGC |
| 31 | CTTA | 76 | TCGT | 121 | TCGC | 166 | TGCT |
| 32 | GGTT | 77 | ACTT | 122 | ACGA | 167 | AACG |
| 33 | TCTT | 78 | GGTA | 123 | CAGT | 168 | CAAA |
| 34 | TTAG | 79 | AACT | 124 | CTCC | 169 | TGAG |
| 35 | ATCT | 80 | TACA | 125 | TCCC | 170 | ACGG |
| 36 | GATA | 81 | CTCG | 126 | TCGA | 171 | TGAA |
| 37 | TATT | 82 | GCTC | 127 | CTGC | 172 | AAGA |
| 38 | ATTA | 83 | AATC | 128 | GCTG | 173 | CACG |
| 39 | GTGA | 84 | GCAT | 129 | TGTC | 174 | GGAT |
| 40 | GACA | 85 | CACT | 130 | CCTG | 175 | TGGA |
| 41 | TTTA | 86 | GTAC | 131 | TACG | 176 | AGTG |
| 42 | AATT | 87 | GCGA | 132 | GTGG | 177 | CCAC |
| 43 | TTCG | 88 | ATCG | 133 | CCTC | 178 | GTCC |
| 44 | GATC | 89 | GTGT | 134 | ACCT | 179 | AATG |
| 45 | ATCA | 90 | CTGT | 135 | TAAG | 180 | GCGG |

<12> The 3' adapter for preparing a nucleic acid library according to <11>, in which the base sequence at the 5'-end is any one selected from the group consisting of the base sequence Nos. 1 to 118 listed in the Table A.

<13> The 3' adapter for preparing a nucleic acid library according to <11> or <12>, in which the nucleotide at the 3'-end of the single-stranded DNA is modified with a compound having a modification structure capable of suppressing binding of nucleic acid.

### Advantageous Effects of Invention

According to the present disclosure, there are provided a method for degrading 5'-adenylated single-stranded DNA, a method for ligating 3' adapter to a nucleic acid; a method for preparing an NGS library, a kit for preparing an NGS library, and a 3' adapter for preparing a nucleic acid library, for enabling rapid and easy preparation of an NGS library, with use of a single-stranded DNA having the nucleotide at the 5'-end that is adenylated, as the 3' adapter.

### BRIEF DESCRIPTION OF DRAWINGS

Fig. 1 is a bar graph showing results of Experiment 1.
Fig. 2 is a photograph of a polyacrylamide gel showing results of Experiment 3.
Fig. 3 is a bar graph showing results of Experiment 4.

### DESCRIPTION OF EMBODIMENTS

Hereinafter, an embodiment of the present disclosure will be described in detail. The present disclosure is however not limited to the following embodiment. In the following disclosure, the components (including element steps and the like) are not essential unless otherwise specified. The same applies to numerical values and ranges thereof, without limiting the present disclosure.

In the present disclosure, each numerical range indicated with use of "to" includes numerical values described before and after "to", respectively as a lower limit value and an upper limit value.

With respect to the numerical ranges described stepwise in the present disclosure, the upper limit value or the lower limit value described in one numerical range may be replaced with the upper limit value or the lower limit value of other numerical range described stepwise. With respect to the numerical ranges described in the present description, the upper limit value or the lower limit value of such numerical ranges may be replaced with values described in Examples.

In a case where a plurality of substances that corresponds to each component is present in a composition, the content rate of each component in the composition in the present disclosure means the total content rate of such plurality of substances present in the composition, unless otherwise specified.

### << Method for Degrading 5'-Adenylated Single-Stranded DNA >>

The method for degrading 5'-adenylated single-stranded DNA of the present disclosure includes degrading the 5'-adenylated single-stranded DNA with RecJ, wherein a base sequence at the 5'-end of the 5'-adenylated single-stranded DNA, exclusive of adenylic acid at the 5'-end, is any one of the base sequences listed in the following Table A:

### [Table 4]

**Table A**

| No. | Base sequence (5' to 3') | No. | Base sequence (5' to 3') | No. | Base sequence (5' to 3') | No. | Base sequence (5' to 3') |
|---|---|---|---|---|---|---|---|
| 1 | GTTA | 46 | TTTC | 91 | TATG | 136 | CCTA |
| 2 | TAAT | 47 | ACAT | 92 | GAGA | 137 | GAGT |
| 3 | GTAT | 48 | GCTA | 93 | TGAT | 138 | AGTA |
| 4 | TGTT | 49 | TCTG | 94 | CTCA | 139 | CGAT |
| 5 | GTTT | 50 | ATTG | 95 | CATA | 140 | CCGT |
| 6 | TTAA | 51 | ATAA | 96 | CTAA | 141 | GGCA |
| 7 | GTTC | 52 | CGTT | 97 | GCAA | 142 | CATG |
| 8 | TACT | 53 | CTTC | 98 | TTGG | 143 | AAGT |
| 9 | CTTT | 54 | GTTG | 99 | CTAG | 144 | ACAA |
| 10 | TCAT | 55 | GTCG | 100 | TAAC | 145 | AGTC |
| 11 | TATA | 56 | CTTG | 101 | TAAA | 146 | CGTA |
| 12 | GATT | 57 | TGTA | 102 | ATCC | 147 | TCCA |
| 13 | TATC | 58 | ACTA | 103 | TCAG | 148 | CCCT |
| 14 | TTTG | 59 | GAAT | 104 | ACTG | 149 | TACC |
| 15 | TTCA | 60 | TCCT | 105 | ATAG | 150 | GGTC |
| 16 | TTAC | 61 | TTTT | 106 | CCTT | 151 | ACCA |
| 17 | TTCT | 62 | GACT | 107 | GAAA | 152 | CGTC |
| 18 | CATT | 63 | TAGT | 108 | CTGA | 153 | TAGG |
| 19 | TCTA | 64 | CAAT | 109 | CCAT | 154 | AAAA |
| 20 | TTGT | 65 | ATTC | 110 | GCGT | 155 | ACAG |
| 21 | TTCC | 66 | TCTC | 111 | TCAA | 156 | CACA |
| 22 | GTCA | 67 | AAAT | 112 | CATC | 157 | ACAC |
| 23 | CTAT | 68 | CTCT | 113 | TTGC | 158 | CGTG |
| 24 | AGTT | 69 | GTAG | 114 | CTAC | 159 | ATGC |
| 25 | ATTT | 70 | ATGT | 115 | ACGT | 160 | TCCG |
| 26 | TTAT | 71 | ATAC | 116 | TCGG | 161 | GCAG |
| 27 | GTCT | 72 | AATA | 117 | ACTC | 162 | ATGG |
| 28 | GTAA | 73 | ATGA | 118 | AACA | 163 | CAGA |
| 29 | ATAT | 74 | TTGA | 119 | TGTG | 164 | TAGA |
| 30 | GCTT | 75 | TCAC | 120 | CTGG | 165 | TAGC |
| 31 | CTTA | 76 | TCGT | 121 | TCGC | 166 | TGCT |
| 32 | GGTT | 77 | ACTT | 122 | ACGA | 167 | AACG |
| 33 | TCTT | 78 | GGTA | 123 | CAGT | 168 | CAAA |
| 34 | TTAG | 79 | AACT | 124 | CTCC | 169 | TGAG |
| 35 | ATCT | 80 | TACA | 125 | TCCC | 170 | ACGG |
| 36 | GATA | 81 | CTCG | 126 | TCGA | 171 | TGAA |
| 37 | TATT | 82 | GCTC | 127 | CTGC | 172 | AAGA |
| 38 | ATTA | 83 | AATC | 128 | GCTG | 173 | CACG |
| 39 | GTGA | 84 | GCAT | 129 | TGTC | 174 | GGAT |
| 40 | GACA | 85 | CACT | 130 | CCTG | 175 | TGGA |
| 41 | TTTA | 86 | GTAC | 131 | TACG | 176 | AGTG |
| 42 | AATT | 87 | GCGA | 132 | GTGG | 177 | CCAC |
| 43 | TTCG | 88 | ATCG | 133 | CCTC | 178 | GTCC |
| 44 | GATC | 89 | GTGT | 134 | ACCT | 179 | AATG |
| 45 | ATCA | 90 | CTGT | 135 | TAAG | 180 | GCGG |

According to the method for degrading 5'-adenylated single-stranded DNA of the present disclosure, it becomes possible to rapidly and easily prepare an NGS library, with use of a single-stranded DNA having the nucleotide at the 5'-end that is adenylated, as the 3' adapter.

Operation of the method for degrading 5'-adenylated single-stranded DNA of the present disclosure, although remaining unclear, is presumed as follows.

In a case where the base sequence at the 5'-end of the 5'-adenylated single-stranded DNA, exclusive of adenylic acid at the 5'-end, is given by any of the specific base sequences listed in the Table A, RecJ recognizes the specific base sequence and comes into contact with the base sequence. RecJ then presumably eliminates nucleotides in the 5' to 3' direction regardless of presence or absence of adenylic acid at the 5'-end, thereby degrading the 5'-adenylated single-stranded DNA.

Note that the present disclosure is by no means limited to such presumed mechanism.

### < 5'-Adenylated Single-Stranded DNA >

In the 5'-adenylated single-stranded DNA of the present disclosure, the base sequence at the 5'-end, exclusive of adenylic acid at the 5'-end, is given by any of base sequence Nos. 1 to 180 listed in the Table A.

The base sequence at the 5'-end, exclusive of adenylic acid at the 5'-end,
is preferably any one selected from the group consisting of the base sequence Nos. 1 to 167 listed in the Table A,
more preferably any one selected from the group consisting of the base sequence Nos. 1 to 157 listed in the Table A,
still more preferably any one selected from the group consisting of the base sequence Nos. 1 to 147 listed in the Table A,
yet more preferably any one selected from the group consisting of the base sequence Nos. 1 to 130 listed in the Table A,
furthermore preferably any one selected from the group consisting of the base sequence Nos. 1 to 118 listed in the Table A,
yet furthermore preferably any one selected from the group consisting of the base sequence Nos. 1 to 100 listed in the Table A,
even more preferably any one selected from the group consisting of the base sequence Nos. 1 to 79 listed in the Table A,
particularly preferably any one selected from the group consisting of the base sequence Nos. 1 to 60 listed in the Table A,
more particularly preferably any one selected from the group consisting of the base sequence Nos. 1 to 40 listed in the Table A,
still more particularly preferably any one selected from the group consisting of the base sequence Nos. 1 to 21 listed in the Table A, and
most preferably any one selected from the group consisting of the base sequence Nos. 1 to 3 listed in the Table A.

Note that the base sequence at the 5'-end of the 5'-adenylated single-stranded DNA, exclusive of adenylic acid at the 5'-end, means a base sequence at the 5'-end assumed for non-adenylated single-stranded DNA.

In the Table A, Nos. 1 to 180 indicating base sequences are assigned in descending order of the relative degradation rate by RecJ. That is, the smaller the number listed in the Table A is, the more likely the 5'-adenylated single-stranded DNA is to be degraded by RecJ.

The relative degradation rate is determined according to a method described in Examples described later.

In one aspect, the relative degradation rate of the 5'-adenylated single-stranded DNA, whose base sequence at the 5'-end, exclusive of adenylic acid at the 5'-end, is given by any of the base sequence Nos. 1 to 180 in the Table A, is 7.286 or more.

In one aspect, the relative degradation rate of the 5'-adenylated single-stranded DNA, whose base sequence at the 5'-end, exclusive of adenylic acid at the 5'-end, is given by any of the base sequence Nos. 1 to 167 in the Table A, is 7.813 or more.

In one aspect, the relative degradation rate of the 5'-adenylated single-stranded DNA, whose base sequence at the 5'-end, exclusive of adenylic acid at the 5'-end, is given by any of the base sequence Nos. 1 to 157 in the Table A, is 8.173 or more.

In one aspect, the relative degradation rate of the 5'-adenylated single-stranded DNA, whose base sequence at the 5'-end, exclusive of adenylic acid at the 5'-end, is given by any of the base sequence Nos. 1 to 147 in the Table A, is 8.489 or more.

In one aspect, the relative degradation rate of the 5'-adenylated single-stranded DNA, whose base sequence at the 5'-end, exclusive of adenylic acid at the 5'-end, is given by any of the base sequence Nos. 1 to 130 in the Table A, is 8.992 or more.

In one aspect, the relative degradation rate of the 5'-adenylated single-stranded DNA, whose base sequence at the 5'-end, exclusive of adenylic acid at the 5'-end, is given by any of the base sequence Nos. 1 to 118 in the Table A, is 9.513 or more.

In one aspect, the relative degradation rate of the 5'-adenylated single-stranded DNA, whose base sequence at the 5'-end, exclusive of adenylic acid at the 5'-end, is given by any of the base sequence Nos. 1 to 100 in the Table A, is 10.464 or more.

In one aspect, the relative degradation rate of the 5'-adenylated single-stranded DNA, whose base sequence at the 5'-end, exclusive of adenylic acid at the 5'-end, is given by any of the base sequence Nos. 1 to 79 in the Table A, is 12.139 or more.

In one aspect, the relative degradation rate of the 5'-adenylated single-stranded DNA, whose base sequence at the 5'-end, exclusive of adenylic acid at the 5'-end, is given by any of the base sequence Nos. 1 to 60 in the Table A, is 13.271 or more.

In one aspect, the relative degradation rate of the 5'-adenylated single-stranded DNA, whose base sequence at the 5'-end, exclusive of adenylic acid at the 5'-end, is given by any of the base sequence Nos. 1 to 40 in the Table A, is 16.494 or more.

In one aspect, the relative degradation rate of the 5'-adenylated single-stranded DNA, whose base sequence at the 5'-end, exclusive of adenylic acid at the 5'-end, is given by any of the base sequence Nos. 1 to 21 in the Table A, is 26.665 or more.

In one aspect, the relative degradation rate of the 5'-adenylated single-stranded DNA, whose base sequence at the 5'-end, exclusive of adenylic acid at the 5'-end, is given by any of the base sequence Nos. 1 to 3 in the Table A, is 43.725 or more.

In the 5'-adenylated single-stranded DNA of the present disclosure, it is also preferred that each of three or four bases among the first to fourth bases in a base sequence at the 5'-end, exclusive of adenylic acid at the 5'-end, is independently given by adenine or thymine.

In the 5'-adenylated single-stranded DNA of the present disclosure, it is also preferred that the base sequence at the 5'-end, exclusive of adenylic acid at the 5'-end, is given by GT (that is, guanine-thymine), or TT (that is, thymine-thymine).

In the 5'-adenylated single-stranded DNA of the present disclosure, the fifth and subsequent bases in the base sequence at the 5'-end, exclusive of adenylic acid at the 5'-end, may follow a freely selectable base sequence.

The fifth and subsequent bases in the base sequence at the 5'-end, exclusive of adenylic acid at the 5'-end, may contain modified base. Examples of the modified base include, but are not limited to, 4-acetylcytidine, dihydrouridine, inosine, and 1-methyladenosine.

The 5'-adenylated single-stranded DNA of the present disclosure preferably has a base length, exclusive of adenylic acid at the 5'-end, of 6 to 1000 bases, more preferably 6 to 100 bases, and still more preferably 10 to 30 bases, from the viewpoint of easy degradation.

The nucleotide at the 3'-end of the 5'-adenylated single-stranded DNA of the present disclosure is preferably modified with a compound having a modified structure capable of suppressing binding of nucleic acid.

Examples of the compound having a modification structure capable of suppressing binding of nucleic acid include dideoxyribonucleoside triphosphate (ddNTP) and amino group.

Examples of dideoxyribonucleoside triphosphate include dideoxycytidine triphosphate (ddCTP), dideoxyguanosine triphosphate (ddGTP), dideoxythymidine triphosphate (ddTTP), and dideoxyadenosine triphosphate (ddATP).

The compound having a modification structure capable of suppressing binding of nucleic acid is preferably dideoxyribonucleoside triphosphate, and more preferably dideoxycytidine triphosphate.

### < RecJ >

RecJ of the present disclosure is not particularly limited as long as it has 5' to 3' exonuclease activity specific to single-stranded DNA.

RecJ of the present disclosure may also be tagged RecJ. Examples of the tag include peptide tag, polypeptide tag, protein tag, and fluorescent protein tag. For example, RecJ tagged with maltose binding protein (MBP) will improve solubility of RecJ, thereby improving the degradation efficiency of the 5'-adenylated single-stranded DNA.

RecJ of the present disclosure may also be post-translationally modified RecJ. Examples of the post-translational modification include phosphorylation, glycosylation, ubiquitination, nitrosylation, methylation, acetylation, acylation, and lipidation.

RecJ of the present disclosure may also be mutant RecJ.

The mutant RecJ is defined to have a sequence identity of, for example, 85% or more, preferably 90% or more, more preferably 95% or more, still more preferably 98% or more, and particularly preferably 99% or more, with the amino acid sequence of wild-type RecJ from which RecJ is derived, and to have 5' to 3' exonuclease activity specific to single-stranded DNA, like the wild-type RecJ.

Examples of methods for calculating the identity of amino acid sequences include known means. For example, the identity can be calculated with use of any of commercially available analysis tools, or analysis tools available through telecommunication line (the Internet). In one example, the identity of amino acid sequence can be calculated according to homology algorithm BLAST (Basic local alignment search tool) http://www.ncbi.nlm.nih.gov/BLAST/ from the National Center for Biotechnology Information (NCBI), with use of default (initially set) parameters.

The mutant RecJ may further be a protein formed of an amino acid sequence of wild-type RecJ from which RecJ is derived, but having therein one or several amino acids substituted, deleted, inserted and/or added, and having 5' to 3' exonuclease activity specific to single-stranded DNA, like the wild-type RecJ. One or several herein can mean, but not restrictively in particular, 1 to 80 for example, preferably 1 to 40, more preferably 1 to 10, still more preferably 1 to 5, and particularly preferably 1 to 3.

RecJ usable herein may be any of commercially available ones, which are exemplified by, but not restrictively, RecJf from New England Biolabs Inc., RecJ Exonuclease from Lucigen Corporation, and the like.

### < Degradation Conditions >

In the method for degrading 5'-adenylated single-stranded DNA of the present disclosure, the degradation conditions are not particularly limited.

Reaction liquid used for degradation may be, for example, a reaction liquid having 5'-adenylated single-stranded DNA, RecJ, and water mixed therein, or may be a reaction liquid that additionally contains metal ion. Examples of the metal ion include magnesium ion, zinc ion, iron ion, copper ion, manganese ion, and calcium ion. The metal ion is preferably magnesium ion.

The reaction liquid may further contain, for example, dimethyl sulfoxide (DMSO), polyethylene glycol (PEG), RNase inhibitor, sodium chloride, tris hydrochloride (Tris-HCl), magnesium chloride, or dithiothreitol (DTT).

The concentration of each substance in the reaction liquid is not particularly limited. It is, however, preferred from the viewpoint of easy degradation of nucleic acid by RecJ that 10 to 200 units of RecJ and 5 to 25 mM of Mg²⁺ are contained with respect to the upper limit of 1 µg of nucleic acid, it is more preferred that 10 to 150 units of RecJ and 5 to 20 mM of Mg²⁺ are contained with respect to the upper limit of 1 µg of nucleic acid, it is still more preferred that 15 to 100 units of RecJ and 6 to 15 mM of Mg²⁺ are contained with respect to the upper limit of 1 µg of nucleic acid, and it is most preferred that 20 to 60 units of RecJ and 8 to 10 mM of Mg²⁺ are contained with respect to 1 µg of nucleic acid. Mg²⁺ herein is preferably contained in the reaction liquid in the form of MgCl₂. In the present disclosure, one unit of RecJ is defined as the amount of enzyme required to produce 0.05 nmol of trichloroacetic acid (TCA)-soluble deoxyribonucleotide, in NEBuffer 2 (New England Biolabs Inc.) that contains 1.5 µg of [³H]-labeled single-stranded DNA from sonicated E. coli, at 37°C for 30 minutes in a total reaction volume of 50 µl.

The degradation temperature is preferably 35°C to 40°C, more preferably 36°C to 38°C, and still more preferably 37°C. The degradation time is preferably 10 to 50 minutes, more preferably 20 to 40 minutes, and still more preferably 30 minutes.

After the degradation of 5'-adenylated single-stranded DNA, the reaction liquid having gone through the degradation reaction may further be heat-treated to inactivate RecJ. For example, the reaction liquid having gone through the degradation reaction may be heat-treated at 50°C to 80°C, or may be heat-treated at 60°C to 70°C, in which heat treatment at 65°C is preferred. The heat treatment time may be, for example, 5 minutes to 40 minutes, or may be 10 minutes to 30 minutes, and is preferably 20 minutes. The heat treatment at 65°C for 20 minutes is particularly preferred.

### < Degradation Activity >

In the present disclosure, the phrase stating that RecJ degrades 5'-adenylated single-stranded DNA means that RecJ degrades 5'-adenylated single-stranded DNA to produce nucleotide.

Degradation of 5'-adenylated single-stranded DNA by RecJ can be confirmed, for example, by electrophoresis or the like. More specifically, the degradation operation for 5'-adenylated single-stranded DNA is conducted according to the aforementioned < Degradation Conditions >, 5'-adenylated single-stranded DNAs before and after degradation are then subjected to electrophoresis, in which the degradation is confirmed by reduced brightness of a band assignable to the 5'-adenylated single-stranded DNA after degradation, as compared with a band assignable to the one before degradation.

### < Other Enzymes >

In a case in which any enzyme other than RecJ is also present in the reaction system of the method for degrading 5'-adenylated single-stranded DNA of the present disclosure, such any enzyme other than RecJ preferably allows the 5'-adenylated single-stranded DNA to remain adenylated. The phrase stating that "any enzyme other than RecJ allows the 5'-adenylated single-stranded DNA to remain adenylated" means that, in a case in which any enzyme other than RecJ is present and RecJ is absent, adenylation of 5'-adenylated single-stranded DNA is maintained. It is therefore preferred that any enzyme other than RecJ does not exert deadenylation activity on 5'-adenylated single-stranded DNA. In the method for degrading 5'-adenylated single-stranded DNA of the present disclosure, only RecJ preferably acts as an enzyme to degrade 5'-adenylated single-stranded DNA. Examples of any enzyme other than RecJ include nucleic acid ligase used for ligation, reverse transcriptase used for reverse transcription reaction, and DNA synthase used for PCR.

### << Method for Ligating 3'Adapter to Nucleic Acid >>

A method for ligating a 3' adapter to a nucleic acid of the present disclosure includes: ligating a 5'-adenylated single-stranded DNA, as the 3' adapter, to the 3'-end of the nucleic acid; and after the ligation of the 5'-adenylated single-stranded DNA, degrading excessive 5'-adenylated single-stranded DNA, by the aforementioned method for degrading the 5'-adenylated single-stranded DNA.

### < 5'-Adenylated Single-Stranded DNA >

Description of 5'-adenylated single-stranded DNA is the same as the description of the 5'-adenylated single-stranded DNA described in the section titled << Method for Degrading 5'-Adenylated Single-Stranded DNA >>, inclusive of definitions, examples, preferred aspects, and the like.

The excessive 5'-adenylated single-stranded DNA herein means 5'-adenylated single-stranded DNA that remained unligated to nucleic acid, despite the operation of ligation to the nucleic acid.

### < Nucleic Acid >

Type of the nucleic acid is not particularly limited.

From the viewpoint of easy ligation with the 5'-adenylated single-stranded DNA, the nucleic acid is preferably single-stranded. In the present disclosure, the nucleic acid may also be, for example, DNA, RNA, analogs thereof, conjugates thereof, or the like. The nucleic acid may also be a small RNA (sRNA). The small RNA may be any of microRNA (miRNA), piRNA, and tsRNA. The nucleic acid may be nucleic acid composed solely of bases A, T, G, C, and U; or is not necessarily nucleic acid composed solely of bases of A, T, G, C, and U. Specific examples of the nucleic acid not composed solely of bases A, T, G, C, and U include nucleic acids having gone through modification such as methylation of DNA or RNA, and editing such as A-to-I RNA editing.

The nucleic acid is also not particularly restricted in terms of origin, and may be naturally-derived nucleic acid (total RNA, for example) or synthetic RNA. The nucleic acid may originate, for example, from biological sample, virus-derived sample, environmental sample, or artificial sample.

Examples of the biological sample include those derived from animal, plant, fungus, bacterium, or the like. Examples of the animal include human and non-human animal. Examples of the non-human animal include non-human mammal (for example, monkey, dog, cat, mouse, rat, rabbit, cow, horse, pig, sheep, and the like); and bird (chicken, quail, and the like).

More specific examples of the animal-derived sample include serum, plasma, whole blood, urine, feces, saliva, bone marrow fluid, lymph fluid, and tissue.

Examples of the environmental sample include those derived from soil, water, and the like.

Examples of the artificial sample include artificial serum, artificial plasma, artificial urine, and the like.

The nucleic acid may be of 1 to 300,000 types, may be of 1 to 10,000 types, and may be of 1 to 2000 types.

The base length of the nucleic acid is not particularly limited, and is preferably 1 to 100 bases long, more preferably 10 to 50 bases long, and still more preferably 16 to 40 bases long.

### < Method for Ligation >

In the method for ligating a 3' adapter to a nucleic acid of the present disclosure, 5'-adenylated single-stranded DNA, as the 3' adapter, is ligated to the 3'-end of the nucleic acid. The 3'-end of the nucleic acid may be ligated with one type of 5'-adenylated single-stranded DNA, or two or more types of 5'-adenylated single-stranded DNA.

In the ligation of the 3'adapter, the method for ligation is not particularly limited. Also enzyme for ligation (i.e. ligase) is not particularly limited, for which any of known enzymes is usable.

Description of 5'-adenylated single-stranded DNA, as the 3' adapter, is the same as the description of the 5'-adenylated single-stranded DNA described in the section titled << Method for Degrading 5'-Adenylated Single-Stranded DNA >>, inclusive of definitions, examples, preferred aspects, and the like.

The method for ligating the 3' adapter to the nucleic acid of the present disclosure may be used for applications such as, for example, preparation of NGS library, preparation of qPCR, and cloning.

### << Method for Preparing NGS Library >>

A method for preparing an NGS library of the present disclosure includes:
ligating 5'-adenylated single-stranded DNA, as a 3' adapter, to each 3'-end of a plurality of types of RNA having different sequences;
after the ligating of the 5'-adenylated single-stranded DNA, degrading excessive 5'-adenylated single-stranded DNA by the aforementioned method for degrading 5'-adenylated single-stranded DNA;
ligating single-stranded RNA, as a 5' adapter, to each 5'-end of the plurality of types of RNA;
synthesizing cDNA from the plurality of types of RNA ligated with the 3' adapter and the 5' adapter by a reverse transcription reaction; and
amplifying the obtained cDNA by PCR.

### < Ligation of 3'Adapter >

In the method for preparing an NGS library of the present disclosure, 5'-adenylated single-stranded DNA, as the 3' adapter, is ligated to each 3'-end of a plurality of types of RNA having different sequences. To the 3'-ends of the plurality of types of RNA, one type of 5'-adenylated single-stranded DNA may be ligated, or two or more types of 5'-adenylated single-stranded DNA may be ligated.

In the ligation of the 3'adapter, the method for ligation is not particularly limited. Also enzyme for ligation (i.e. ligase) is not particularly limited, for which any of known enzymes is usable.

Description of 5'-adenylated single-stranded DNA, as the 3' adapter, is the same as the description of the 5'-adenylated single-stranded DNA described in the section titled << Method for Degrading 5'-Adenylated Single-Stranded DNA >>, inclusive of definitions, examples, preferred aspects, and the like.

Types of the plurality of types of RNA are not particularly limited.

From the viewpoint of easy ligation with the 5'-adenylated single-stranded DNA, the RNA is preferably single-stranded. In the present disclosure, the RNA may also be, for example, RNA analog, conjugate of RNA with the RNA analog, or the like. The RNA may also be a small RNA (sRNA). The small RNA may be any of microRNA (miRNA), piRNA, and tsRNA. The plurality of types of RNA may be RNA composed solely of bases A, G, C, and U; or are not necessarily RNA composed solely of bases of A, G, C, and U. Specific examples of the nucleic acid not composed solely of bases A, T, G, C, and U include RNA having gone through modification such as methylation; or RNA having gone through editing such as A-to-I RNA editing.

The RNA is also not particularly restricted in terms of origin, and may be naturally-derived RNA (total RNA, for example) or synthetic RNA. The RNA may originate, for example, from biological sample, virus-derived sample, environmental sample, or artificial sample.

Examples of the biological sample include those derived from animal, plant, fungus, bacterium, or the like. Examples of the animal include human and non-human animal. Examples of the non-human animal include non-human mammal (for example, monkey, dog, cat, mouse, rat, rabbit, cow, horse, pig, sheep, and the like); and bird (chicken, quail, and the like).

More specific examples of the animal-derived sample include serum, plasma, whole blood, urine, feces, saliva, bone marrow fluid, lymph fluid, and tissue.

Examples of the environmental sample include those derived from soil, water, and the like.

Examples of the artificial sample include artificial serum, artificial plasma, artificial urine, and the like.

The plurality of types of RNA may be of 1 to 300,000 types, may be of 1 to 10,000 types, and may be of 1 to 2000 types.

The base length of the plurality of types of RNA is not particularly limited, and is preferably 1 to 100 bases long, more preferably 10 to 50 bases long, and still more preferably 16 to 40 bases long.

### < Method for Degrading Excessive 5'-Adenylated Single-Stranded DNA >

In the method for preparing an NGS library of the present disclosure, excessive 5'-adenylated single-stranded DNA is degraded by the aforementioned method for degrading 5'-adenylated single-stranded DNA, after ligation of 5'-adenylated single-stranded DNA.

The excessive 5'-adenylated single-stranded DNA herein means 5'-adenylated single-stranded DNA that remained unligated to nucleic acid, despite the operation of ligation to the nucleic acid.

Description of degradation herein is the same as the description of the method for degrading 5'-adenylated single-stranded DNA described in the section titled << Method for Degrading 5'-Adenylated Single-Stranded DNA >>, inclusive of definitions, examples, preferred aspects, and the like.

In a case in which any enzyme other than RecJ is also present in the reaction system of degradation of 5'-adenylated single-stranded DNA, such any enzyme other than RecJ preferably allows the 5'-adenylated single-stranded DNA to remain adenylated. It is therefore preferred that any enzyme other than RecJ does not exert deadenylation activity on 5'-adenylated single-stranded DNA. In the method for degrading 5'-adenylated single-stranded DNA of the present disclosure, only RecJ preferably acts as an enzyme to degrade 5'-adenylated single-stranded DNA.

### < Ligation of 5'Adapter >

In the method for preparing an NGS library of the present disclosure, single-stranded RNA, as the 5' adapter, is ligated to each 5'-end of the plurality of types of RNA. To the 5'-ends of the plurality of types of RNA, one type of 5' adapter may be ligated, or two or more types of 5' adapter may be ligated.

Other characteristics of the 5' adapter are not particularly limited, as long as the 5' adapter is single-stranded RNA. Also the base length of the single-stranded RNA as the 5' adapter is not particularly limited, and is preferably 5 to 100 bases long, more preferably 15 to 50 bases long, and still more preferably 20 to 40 bases long.

In the ligation of the 5'adapter, the method for ligation is not particularly limited. Also enzyme for ligation (i.e. ligase) is not particularly limited, for which any of known enzymes is usable.

### < Synthesis of cDNA >

In the method for preparing an NGS library of the present disclosure, cDNA is synthesized from the plurality of types of RNA ligated with the 3' adapter and the 5' adapter by a reverse transcription reaction.

In one aspect, the reverse transcription reaction includes annealing a reverse transcription primer, to the plurality of types of RNA ligated with the 3' adapter and the 5' adapter. The reverse transcription primer may have a sequence that further adds a unique sequence to each type of RNA. Examples of the unique sequence include Unique molecular identifier (UMI) sequence. By attaching a sequence unique to each type of RNA to cDNA, the abundance of each RNA having been present before amplification can be determined even after cDNA is amplified by PCR as described later.

Method of reverse transcription reaction for synthesizing cDNA is not particularly limited. For example, also reverse transcriptase, conditions for reverse transcription reaction, and the like may be any of known ones. For example, the reverse transcription reaction can be conducted by reverse transcription of the plurality of types of RNA ligated with the 3' adapter and the 5' adapter, in the presence of reverse transcriptase, primer, dNTP mix, and an appropriate buffer.

### < Amplification of cDNA >

In the method for preparing an NGS library of the present disclosure, the obtained cDNA is amplified by PCR.

Method of PCR is not particularly limited. For example, any of known PCR enzymes, PCR conditions, and the like is usable.

### < Order of Individual Steps >

A method for preparing an NGS library of the present disclosure includes:
ligating 5'-adenylated single-stranded DNA, as the 3' adapter, to each 3'-end of a plurality of types of RNA having different sequences;
after the ligating of the 5'-adenylated single-stranded DNA, degrading excessive 5'-adenylated single-stranded DNA by the aforementioned method for degrading 5'-adenylated single-stranded DNA;
ligating single-stranded RNA, as the 5' adapter, to each 5'-end of the plurality of types of RNA;
synthesizing cDNA from the plurality of types of RNA ligated with the 3' adapter and the 5' adapter by a reverse transcription reaction; and
amplifying the obtained cDNA by PCR, all of which are preferably conducted in this order.

### << Kit for Preparing NGS Library >>

The kit for preparing an NGS library of the present disclosure includes: 5'-adenylated single-stranded DNA stored in a first container; and RecJ stored in a second container, wherein a base sequence at the 5'-end of the 5'-adenylated single-stranded DNA, exclusive of adenylic acid at the 5'-end, is any one of the base sequences listed in the Table A.

### < First Container >

In the first container of the present disclosure, 5'-adenylated single-stranded DNA is stored. Note that the description of 5'-adenylated single-stranded DNA is the same as the description of the 5'-adenylated single-stranded DNA described in the section titled << Method for Degrading 5'-Adenylated Single-Stranded DNA >>, inclusive of definitions, examples, preferred aspects, and the like.

The first container may also store a buffer for stably keeping the 5'-adenylated single-stranded DNA. Examples of components contained in the buffer include tris hydrochloric acid (Tris-HCl) and ethylenediaminetetraacetic acid (EDTA).

### < Second Container >

In the second container of the present disclosure, RecJ is stored. Note that the description of RecJ is the same as the description of RecJ described in the section titled << Method for Degrading 5'-Adenylated Single-Stranded DNA >>, inclusive of definitions, examples, preferred aspects, and the like.

The second container may also store a buffer for stably keeping RecJ. Examples of components contained in the buffer include tris hydrochloric acid (Tris-HCl), Triton (registered trademark) X, potassium chloride, sodium chloride, dithiothreitol (DTT), ethylenediaminetetraacetic acid (EDTA), bovine serum albumin (BSA), and glycerol.

### < Other Containers >

For the kit for preparing an NGS library of the present disclosure, it is further preferred that at least one selected from the group consisting of 5' adapter, ligase, reverse transcriptase, reverse transcription primer, PCR enzyme, and PCR primer is contained, in a manner such that each is stored in a separate container.

The 5' adapter is preferably single-stranded RNA. Other characteristics of the 5' adapter are not particularly limited, allowing free setting, for example, of the base sequence, the base length, and the like of the 5' adapter.

The ligase, the reverse transcriptase, the reverse transcription primer, the PCR enzyme, and the PCR primer may be any of known ones.

### << 3'Adapter for Preparing Nucleic Acid Library >>

The 3' adapter for preparing a nucleic acid library of the present disclosure is a single-stranded DNA having the nucleotide at the 5'-end that is adenylated, and a base sequence at the 5'-end of the single-stranded DNA, exclusive of adenylic acid at the 5'-end, being any one of the base sequences listed in the Table A.

Note that the description of 3' adapter for preparing a nucleic acid library is the same as the description of the 5'-adenylated single-stranded DNA described in the section titled << Method for Degrading 5'-Adenylated Single-Stranded DNA >>, inclusive of definitions, examples, preferred aspects, and the like.

The 3' adapter for preparing a nucleic acid library can prepare the nucleic acid library, for example by addition to the 3'-end of nucleic acid.

### EXAMPLES

Hereinafter, the present disclosure will be more specifically described with reference to Examples, without limiting the present disclosure to such Examples below, as far as it does not go beyond the gist thereof. Note that "%" is given on a mass basis unless otherwise specified.

In Examples of the present disclosure, a method for preparing an NGS library was conducted referring to Seda Eminaga et al., Quantification of microRNA Expression with Next-Generation Sequencing, Curr Protoc Mol Biol. 2013 July; 04: Unit 4.17., unless otherwise specified.

### << Experiment 1 >>

[1] Ligation of 3'Adapter
   Nuclease free water 1.0 µL
   T4 RNA Ligase Reaction Buffer (NEB/M0242) 1.0 µL
   100% DMSO (13445-74/Nacalai Tesque) 1.0 µL
   50% PEG (NEB/B1004) 1.0 µL
   Total RNA (human serum-derived RNA) 4.0 µL
   To the mixes above, 10 µM of 3' adapter was added, followed by incubation at 90°C for 30 seconds.

The 3' adapter is the "5'-adenylated single-stranded DNA" in the present disclosure. The 3' adapter is a purchased synthetic oligonucleotide, whose base sequence is given by SEQ ID NO: 9 below. The 3' adapter is adenylated at the 5'-end, and blocked by dideoxycytidine modification at the 3'-end.
[SEQ ID NO: 9]5'-App/AACTGTAGGCACCATCAAT/3'ddC

The total RNA is the "nucleic acid" described in << Method for Ligating 3'Adapter to Nucleic Acid >> of the present disclosure, or "a plurality of types of RNA" described in << Method for Preparing NGS Library >> of the present disclosure.

T4 RNA Ligase 2, truncated (NEB/M0242) 1.5 µL

SUPERase inhibitor (Thermo Fisher/AM2694) 0.5 µL

The mixes above were further added, and the mixture was incubated at 22°C for 60 minutes to ligate the total RNA and the 3' adapter.

### Degradation of Excessive 3'Adapter

The solution obtained by the operation in [1] above was further subjected to the following operations under condition 1 to condition 3 below.

### (Condition 1)

Nothing was added to the solution obtained by the operation in [1] above.

### (Condition 2)

To the solution obtained by the operation in [1] above, 2 µL of 5' Deadenylase (NEB/M0331S) as a deadenylase was added, followed by incubation at 30°C for one hour. Thereafter, the following mixes were added, and the mixture was incubated at 37°C for 30 minutes, and further incubated at 65°C for 20 minutes.
Nuclease free water 1.9 µL
5M NaCl 0.1 µL
RecJf (NEB/M0264L) 2.0 µL

### (Condition 3)

To the solution obtained by the operation in [1] above and left untreated with the deadenylase, the mixes described in (Condition 2) were added and incubated at 37°C for 30 minutes, and further incubated at 65°C for 20 minutes.

### Ligation of 5'Adapter

Nuclease free water 0.8 µL
ATP (NEB/0204M) 1.4 µL
T4 RNA Ligase 1 (NEB/0204M) 1.5 µL

To the solution obtained by the operation in [2] above, the mixes above, as well as 10 µM of 5' adapter were added, and the mixture was incubated at 20°C for 60 minutes.

The base sequence of the 5' adapter is given by SEQ ID NO: 1 below.
[SEQ ID NO: 1]5'-rGrUrUrCrArGrArGrUrUrCrUrArCrArGrUrCrCrGrArCrGrArUrC-3'

### Reverse Transcription

To the solution obtained by the operation in [3] above, 1 µL of 10 µM RT primer was added, followed by incubation at 90°C for 30 seconds, and further by incubation at 65°C for 5 minutes.
Nuclease free water 3.5 µL
25 mM dNTP mix (NIPPON GENE/312-07271) 0.3 µL
5×FS Buffer (Thermo Fisher/18080093) 1.5 µL
0.1 M DTT (Thermo Fisher/18080093) 1.5 µL
RNase OUT (Thermo Fisher/10777019) 0.375 µL
SuperScript III (Thermo Fisher/18080093) 1.5 µL

To 7.5 µL of the solution thus obtained by incubation, the mixes above were further added, and the mixture was incubated at 48°C for 30 seconds, and further incubated at 85°C for 5 minutes.

The base sequence of the RT primer is given by SEQ ID NO: 2 below. A moiety indicated by N represents a UMI sequence. In SEQ ID NO:2, each N independently represents A, T, G or C.

[SEQ ID NO: 2]5'-
GTGACTGGAGTTCAGACGTGTGCTCTTCCGATCTNNNNNNNNNNNNATTGATGGT
GCCTACAGTT-3'

### PCR

Nuclease free water 24.1 µL
Phusion HF Buffer (NEB/M0530L) 10 µL
25 mM dNTP mix (NIPPON GENE/312-07271) 0.4 µL
Phusion High-Fidelity DNA Polymerase (NEB/M0530L) 0.5 µL
10 µM PCR primer R 2.5 µL
10 µM PCR primer F 2.5 µL

To 10 µL of the solution obtained by the operation in [4] above, the mixes above were added, the mixture was then incubated at 98°C for 30 seconds, followed by PCR that involves 18 cycles, each cycle consisting of 98°C for 10 seconds, 60°C for 20 seconds, and 72°C for 20 seconds.

The base sequence of the PCR primer R is given by SEQ ID NO: 3 below.
[SEQ ID NO: 3]5'-CAAGCAGAAGACGGCATACGAGATCGTGATGCTCCACCGATAAATATTAGCCCGT-3'

The base sequence of the PCR primer F is given by SEQ ID NO: 4 below.
[SEQ ID NO: 4]5'-AATGATACGGCGACCACCGAGATCTACACGTTCAGAGTTCTACAGTCCGA-3'

### Sequencing

The solution obtained by the operations up to [5] above was purified with use of AMPure XP (Beckman Coulter/A63881), to complete the preparation of the NGS library. The completed library was sequenced on Illumina Nextseq550, and data was analyzed by QIAGEN GeneGlobe, to map known microRNA sequences.

### [Results]

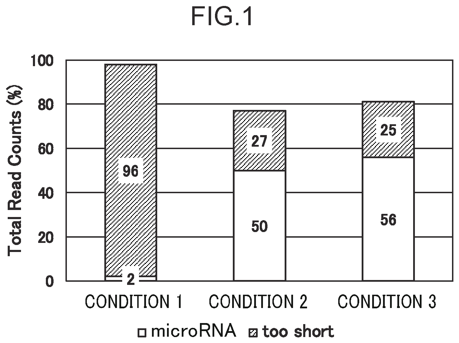

Fig. 1 shows the rate (%) of microRNA read counts, and the rate (%) of too short read counts, relative to the total read counts.

The rate of microRNA read counts means proportion of reads whose sequences are same as or almost the same as the sequences on the miRBase database, among the total reads. The rate of the too short read counts means proportion of reads having the 3' adapter and the 5' adapter bound thereto, and reads having a short sequence of 1 to 15 bases between the 3'adapter and the 5' adapter, among the total reads.

Accordingly, it was demonstrated that the excessive 3' adapter was degradable with use of RecJ only, even in the absence of deadenylase, and also that preparation of the NGS library was feasible.

### << Experiment 2 >>

5'-Adenylated single-stranded DNA, having a base sequence at the 5'-end exclusive of adenylic acid at the 5'-end, in which the first to fourth bases are randomized (also referred to as 5'-randomized DNA, hereinafter, the base sequence is given by SEQ ID NO:6) was purchased. Note that the 3'-end of the 5'-randomized DNA is blocked by dideoxycytidine modification.
[SEQ ID NO: 6]5'-App/NNNNGTAGGCACCATCAAT/3'ddC

In SEQ ID NO: 6, each N independently represents A, T, G, or C. That is, 256 (= 4⁴) types of 5'-randomized DNA were obtained.

Each 5'-randomized DNA was treated with RecJ as follows.
(RecJ-Treated Group)
Nuclease free water 4.3 µL
T4 RNA Ligase Reaction Buffer (NEB/B0216L) 1 µL
100% DMSO (13445-74/Nacalai Tesque) 1 µL
50% PEG (NEB/B1004) 1 µL
500 mM NaCl (31334-51/Nacalai Tesque) 1.2 µL
SUPERase inhibitor (AM2696/Thermo) 0.5 µL
RecJf (M0264S/NEB) 2 µL
5 µM 5'-randomized DNA 1 µL

The solution above was incubated at 37°C for 30 minutes, and further incubated at 65°C for 20 minutes, after which the 5'-randomized DNA was purified with use of QIAquick Nucleotide Removal Kit (28306/QIAGEN). That is, 5'-randomized DNA that remained undegraded by RecJ was obtained in a purified form.

### (Control Group)

The aforementioned solution in (RecJ-Treated Group) above was incubated in the same manner as for the (RecJ-Treated Group) above, except that RecJf was replaced by Nuclease free water, and 5'-randomized DNA was purified.

With use of the 5'-randomized DNA obtained by purification in (RecJ-Treated Group) and (Control Group) as the 3' adapter, the NGS library was prepared by subjecting the total RNA to be contained in the library. Note as described previously, method for preparing the NGS library was conducted referring to Seda Eminaga et al., Quantification of microRNA Expression with Next-Generation Sequencing, Curr Protoc Mol Biol. 2013 July; 04: Unit 4.17., unless otherwise specified. More specifically, [1] Ligation of 3' Adapter, annealing of primer for reverse transcription, [3] Ligation of 5' Adapter, [4] Reverse Transcription, and [5] PCR described in << Experiment 1>> were conducted in this order.

The base sequence of the RT primer is given by SEQ ID NO: 5 below. A moiety indicated by N represents a UMI sequence. In SEQ ID NO: 5, each N independently represents A, T, G, or C.

[SEQ ID NO: 5]5'-
GTGACTGGAGTTCAGACGTGTGCTCTTCCGATCTNNNNNNNNNNNNATTGATGGT
GCCTAC-3'

Furthermore, NNNN moiety of the 3' adapters was analyzed for (RecJ-Treated Group) and (Control Group). More specifically, a read count value was acquired for each sequence of the randomized base sequence moiety (NNNN), then subjected to Reads Per Million mapped reads(RPM) correction, thereby determining degradation rate of the RecJ-treated group relative to the control group. The base sequences were ranked from No. 1 in descending order of the degradation rate, to obtain base sequences Nos. 1 to 256.

Log₂(RPM) equivalent value of the read count was determined by expression (1) below.

Log₂((Read count of each sequence/Total read counts of all 256 types)*1,000,000)) Expression (1)

The degradation rate was determined by expression (2) below. 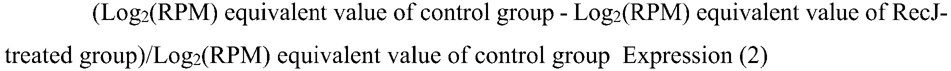 (Log2(RPM) equivalent value of control group - Log2(RPM) equivalent value of RecJ-treated group)/Log2(RPM) equivalent value of control group Expression (2)

Assuming now the relative degradation rate of the base sequence No. 256, that demonstrated the lowest degradation rate among the base sequences Nos. 1 to 256, as 0.000, the relative degradation rate was determined for each of the base sequences Nos. 1 to 255.

### [Results]

Results are shown in Tables 5, 6, 7, and 8.

From the results, RecJ was found to degrade the 5'-adenylated single-stranded DNA without deadenylation by any enzyme other than RecJ, in a case where the 5'-adenylated single-stranded DNA has specific base sequences, in the base sequence at the 5'-end exclusive of adenylic acid at the 5'-end.

Referring to Table 6, the 5'-adenylated single-stranded DNA used in << Experiment 1 >> was found to be ranked 79th out of 256. Referring to Table 8, the 5'-adenylated single-stranded DNA, used in Patent Document 1 (WO 2011/056866 A) and in need of deadenylase for degrading the 3' adapter, was found to be ranked 201st out of 256.

**[Table 5]**

| No. | Base sequence (5' to 3') | Log₂(RPM) equivalent value of read count | | Relative decomposition rate | No. | Base sequence (5' to 3') | Log₂(RPM) equivalent value of read count | | Relative decomposition rate |
|---|---|---|---|---|---|---|---|---|---|
| | | RecJ-treated group | Control group | | | | RecJ-treated group | Control group | |
| 1 | GTTA | 6.54 | 10.39 | 46.412 | 33 | TCTT | 5.93 | 6.62 | 19.733 |
| 2 | TAAT | 5.23 | 8.04 | 44.312 | 34 | TTAG | 9.15 | 10.15 | 19.210 |
| 3 | GTAT | 6.36 | 9.70 | 43.725 | 35 | ATCT | 9.25 | 10.26 | 19.196 |
| 4 | TGTT | 5.90 | 8.79 | 42.234 | 36 | GATA | 9.74 | 10.74 | 18.632 |
| 5 | GTTT | 5.47 | 7.99 | 40.913 | 37 | TATT | 5.58 | 6.07 | 17.417 |
| 6 | TTAA | 6.03 | 8.70 | 40.024 | 38 | ATTA | 8.22 | 8.92 | 17.208 |
| 7 | GTTC | 7.27 | 10.16 | 37.861 | 39 | GTGA | 10.58 | 11.40 | 16.539 |
| 8 | TACT | 6.38 | 8.91 | 37.793 | 40 | GACA | 8.31 | 8.95 | 16.494 |
| 9 | CTTT | 8.90 | 12.35 | 37.310 | 41 | TTTA | 6.54 | 7.03 | 16.308 |
| 10 | TCAT | 6.58 | 9.12 | 37.138 | 42 | AATT | 7.39 | 7.92 | 16.044 |
| 11 | TATA | 6.60 | 9.13 | 36.990 | 43 | TTCG | 9.92 | 10.64 | 16.044 |
| 12 | GATT | 6.84 | 9.42 | 36.770 | 44 | GATC | 10.14 | 10.86 | 15.958 |
| 13 | TATC | 6.68 | 9.16 | 36.430 | 45 | ATCA | 10.91 | 11.65 | 15.697 |
| 14 | TTTG | 7.96 | 10.91 | 36.416 | 46 | TTTC | 6.47 | 6.91 | 15.621 |
| 15 | TTCA | 6.68 | 8.99 | 34.975 | 47 | ACAT | 9.24 | 9.84 | 15.522 |
| 16 | TTAC | 7.01 | 9.41 | 34.881 | 48 | GCTA | 10.93 | 11.65 | 15.504 |
| 17 | TTCT | 6.59 | 8.79 | 34.332 | 49 | TCTG | 11.15 | 11.79 | 14.801 |
| 18 | CATT | 8.86 | 11.80 | 34.264 | 50 | ATTG | 11.20 | 11.83 | 14.639 |
| 19 | TCTA | 7.26 | 9.35 | 31.756 | 51 | ATAA | 10.36 | 10.93 | 14.565 |
| 20 | TTGT | 9.22 | 11.50 | 29.195 | 52 | CGTT | 11.39 | 11.99 | 14.339 |
| 21 | TTCC | 6.93 | 8.39 | 26.665 | 53 | CTTC | 12.96 | 13.62 | 14.227 |
| 22 | GTCA | 9.05 | 10.62 | 24.086 | 54 | GTTG | 11.88 | 12.47 | 14.091 |
| 23 | CTAT | 11.25 | 13.04 | 23.086 | 55 | GTCG | 10.30 | 10.81 | 14.086 |
| 24 | AGTT | 7.51 | 8.57 | 21.784 | 56 | CTTG | 12.34 | 12.93 | 13.904 |
| 25 | ATTT | 6.94 | 7.91 | 21.588 | 57 | TGTA | 9.72 | 10.17 | 13.817 |
| 26 | TTAT | 5.65 | 6.39 | 20.901 | 58 | ACTA | 9.72 | 10.17 | 13.809 |
| 27 | GTCT | 10.11 | 11.40 | 20.680 | 59 | GAAT | 8.89 | 9.25 | 13.304 |
| 28 | GTAA | 9.28 | 10.45 | 20.574 | 60 | TCCT | 10.09 | 10.50 | 13.271 |
| 29 | ATAT | 7.56 | 8.50 | 20.446 | 61 | TTTT | 6.68 | 6.95 | 13.205 |
| 30 | GCTT | 10.30 | 11.53 | 20.064 | 62 | GACT | 9.98 | 10.37 | 13.096 |
| 31 | CTTA | 11.79 | 13.18 | 19.882 | 63 | TAGT | 10.09 | 10.48 | 13.091 |
| 32 | GGTT | 8.32 | 9.28 | 19.759 | 64 | CAAT | 11.55 | 11.97 | 12.890 |

**[Table 6]**

| No. | Base sequence (5' to 3') | Log₂(RPM) equivalent value of read count | | Relative decomposition rate | No. | Base sequence (5' to 3') | Log₂(RPM) equivalent value of read count | | Relative decomposition rate |
|---|---|---|---|---|---|---|---|---|---|
| | | RecJ-treated group | Control group | | | | RecJ-treated group | Control group | |
| 65 | ATTC | 8.74 | 9.05 | 12.868 | 97 | GCAA | 9.77 | 9.90 | 10.697 |
| 66 | TCTC | 6.07 | 6.29 | 12.851 | 98 | TTGG | 11.45 | 11.59 | 10.513 |
| 67 | AAAT | 8.74 | 9.05 | 12.839 | 99 | CTAG | 12.79 | 12.94 | 10.490 |
| 68 | CTCT | 13.33 | 13.81 | 12.800 | 100 | TAAC | 9.24 | 9.35 | 10.464 |
| 69 | GTAG | 10.50 | 10.87 | 12.799 | 101 | TAAA | 8.34 | 8.43 | 10.459 |
| 70 | ATGT | 12.71 | 13.10 | 12.366 | 102 | ATCC | 10.74 | 10.85 | 10.440 |
| 71 | ATAC | 11.06 | 11.40 | 12.345 | 103 | TCAG | 9.38 | 9.48 | 10.388 |
| 72 | AATA | 9.91 | 10.21 | 12.326 | 104 | ACTG | 11.71 | 11.82 | 10.331 |
| 73 | ATGA | 12.12 | 12.49 | 12.325 | 105 | ATAG | 11.73 | 11.85 | 10.323 |
| 74 | TTGA | 10.75 | 11.08 | 12.324 | 106 | CCTT | 13.47 | 13.60 | 10.320 |
| 75 | TCAC | 8.92 | 9.18 | 12.220 | 107 | GAAA | 8.78 | 8.86 | 10.211 |
| 76 | TCGT | 10.87 | 11.19 | 12.172 | 108 | CTGA | 13.54 | 13.62 | 9.971 |
| 77 | ACTT | 7.52 | 7.74 | 12.170 | 109 | CCAT | 12.75 | 12.80 | 9.825 |
| 78 | GGTA | 10.79 | 11.10 | 12.154 | 110 | GCGT | 11.94 | 11.99 | 9.822 |
| 79 | AACT | 9.17 | 9.43 | 12.139 | 111 | TCAA | 9.35 | 9.39 | 9.762 |
| 80 | TACA | 8.23 | 8.46 | 12.084 | 112 | CATC | 13.11 | 13.17 | 9.751 |
| 81 | CTCG | 11.63 | 11.94 | 11.944 | 113 | TTGC | 12.30 | 12.35 | 9.751 |
| 82 | GCTC | 10.95 | 11.24 | 11.944 | 114 | CTAC | 13.89 | 13.94 | 9.709 |
| 83 | AATC | 10.70 | 10.98 | 11.913 | 115 | ACGT | 11.78 | 11.81 | 9.596 |
| 84 | GCAT | 10.99 | 11.26 | 11.712 | 116 | TCGG | 10.21 | 10.24 | 9.590 |
| 85 | CACT | 11.62 | 11.89 | 11.653 | 117 | ACTC | 8.31 | 8.33 | 9.583 |
| 86 | GTAC | 10.80 | 11.05 | 11.651 | 118 | AACA | 9.55 | 9.56 | 9.513 |
| 87 | GCGA | 10.03 | 10.26 | 11.648 | 119 | TGTG | 11.57 | 11.58 | 9.442 |
| 88 | ATCG | 12.19 | 12.46 | 11.522 | 120 | CTGG | 13.32 | 13.31 | 9.309 |
| 89 | GTGT | 12.88 | 13.14 | 11.302 | 121 | TCGC | 10.01 | 10.00 | 9.289 |
| 90 | CTGT | 14.38 | 14.64 | 11.097 | 122 | ACGA | 10.50 | 10.49 | 9.277 |
| 91 | TATG | 11.40 | 11.60 | 11.091 | 123 | CAGT | 12.88 | 12.87 | 9.263 |
| 92 | GAGA | 8.74 | 8.89 | 11.034 | 124 | CTCC | 13.58 | 13.56 | 9.255 |
| 93 | TGAT | 9.36 | 9.52 | 11.004 | 125 | TCCC | 9.47 | 9.45 | 9.215 |
| 94 | CTCA | 13.35 | 13.55 | 10.817 | 126 | TCGA | 10.09 | 10.07 | 9.139 |
| 95 | CATA | 12.67 | 12.85 | 10.731 | 127 | CTGC | 13.97 | 13.92 | 9.032 |
| 96 | CTAA | 13.22 | 13.40 | 10.712 | 128 | GCTG | 12.28 | 12.24 | 9.027 |

**[Table 7]**

| No. | Base sequence (5' to 3') | Log₂(RPM) equivalent value of read count | | Relative decomposition rate | No. | Base sequence (5' to 3') | Log₂(RPM) equivalent value of read count | | Relative decomposition rate |
|---|---|---|---|---|---|---|---|---|---|
| | | RecJ-treated group | Control group | | | | RecJ-treated group | Control group | |
| 129 | TGTC | 12.57 | 12.52 | 9.016 | 161 | GCAG | 9.86 | 9.73 | 8.000 |
| 130 | CCTG | 12.85 | 12.81 | 8.992 | 162 | ATGG | 12.87 | 12.69 | 7.973 |
| 131 | TACG | 9.34 | 9.31 | 8.991 | 163 | CAGA | 11.09 | 10.94 | 7.954 |
| 132 | GTGG | 11.35 | 11.31 | 8.974 | 164 | TAGA | 9.50 | 9.37 | 7.949 |
| 133 | CCTC | 12.85 | 12.79 | 8.952 | 165 | TAGC | 10.34 | 10.20 | 7.938 |
| 134 | ACCT | 11.39 | 11.34 | 8.946 | 166 | TGCT | 11.12 | 10.96 | 7.914 |
| 135 | TAAG | 8.89 | 8.85 | 8.910 | 167 | AACG | 9.20 | 9.06 | 7.813 |
| 136 | CCTA | 13.29 | 13.23 | 8.907 | 168 | CAAA | 12.05 | 11.87 | 7.784 |
| 137 | GAGT | 10.95 | 10.90 | 8.871 | 169 | TGAG | 8.82 | 8.68 | 7.766 |
| 138 | AGTA | 10.04 | 10.00 | 8.864 | 170 | ACGG | 10.82 | 10.65 | 7.719 |
| 139 | CGAT | 12.06 | 11.98 | 8.756 | 171 | TGAA | 8.88 | 8.73 | 7.692 |
| 140 | CCGT | 13.12 | 13.04 | 8.755 | 172 | AAGA | 9.51 | 9.36 | 7.680 |
| 141 | GGCA | 9.12 | 9.06 | 8.687 | 173 | CACG | 11.25 | 11.05 | 7.612 |
| 142 | CATG | 13.07 | 12.98 | 8.641 | 174 | GGAT | 10.10 | 9.93 | 7.611 |
| 143 | AAGT | 10.59 | 10.51 | 8.613 | 175 | TGGA | 8.74 | 8.59 | 7.603 |
| 144 | ACAA | 10.28 | 10.20 | 8.574 | 176 | AGTG | 10.49 | 10.29 | 7.464 |
| 145 | AGTC | 13.36 | 13.25 | 8.557 | 177 | CCAC | 11.78 | 11.56 | 7.434 |
| 146 | CGTA | 12.75 | 12.64 | 8.515 | 178 | GTCC | 10.70 | 10.50 | 7.429 |
| 147 | TCCA | 9.56 | 9.48 | 8.489 | 179 | AATG | 11.24 | 11.01 | 7.300 |
| 148 | CCCT | 12.61 | 12.49 | 8.425 | 180 | GCGG | 10.72 | 10.51 | 7.286 |
| 149 | TACC | 9.67 | 9.58 | 8.419 | 181 | CAAC | 12.36 | 12.11 | 7.277 |
| 150 | GGTC | 11.53 | 11.42 | 8.404 | 182 | CCAA | 11.82 | 11.58 | 7.263 |
| 151 | ACCA | 10.18 | 10.08 | 8.369 | 183 | GCAC | 10.19 | 9.98 | 7.247 |
| 152 | CGTC | 13.90 | 13.77 | 8.357 | 184 | CCAG | 11.43 | 11.19 | 7.230 |
| 153 | TAGG | 9.63 | 9.54 | 8.344 | 185 | CCGG | 11.94 | 11.69 | 7.212 |
| 154 | AAAA | 9.78 | 9.68 | 8.313 | 186 | CAAG | 11.56 | 11.31 | 7.183 |
| 155 | ACAG | 9.86 | 9.75 | 8.239 | 187 | GAAC | 9.86 | 9.64 | 7.123 |
| 156 | CACA | 11.69 | 11.56 | 8.210 | 188 | CCGA | 12.00 | 11.73 | 7.004 |
| 157 | ACAC | 10.28 | 10.16 | 8.173 | 189 | AGAT | 9.02 | 8.81 | 7.000 |
| 158 | CGTG | 12.30 | 12.15 | 8.133 | 190 | CCCG | 10.86 | 10.61 | 6.976 |
| 159 | ATGC | 13.14 | 12.98 | 8.076 | 191 | TGCG | 9.55 | 9.33 | 6.975 |
| 160 | TCCG | 9.69 | 9.56 | 8.031 | 192 | AAAC | 10.13 | 9.88 | 6.866 |

**[Table 8]**

| No. | Base sequence (5' to 3') | Log₂(RPM) equivalent value of read count | | Relative decomposition rate | No. | Base sequence (5' to 3') | Log₂(RPM) equivalent value of read count | | Relative decomposition rate |
|---|---|---|---|---|---|---|---|---|---|
| | | RecJ-treated group | Control group | | | | RecJ-treated group | Control group | |
| 193 | TGCA | 9.57 | 9.33 | 6.824 | 225 | CCCC | 11.48 | 11.06 | 5.597 |
| 194 | GGGA | 9.17 | 8.94 | 6.817 | 226 | CGCG | 10.95 | 10.55 | 5.576 |
| 195 | CAGG | 11.59 | 11.30 | 6.809 | 227 | CAGC | 12.50 | 12.05 | 5.552 |
| 196 | CCCA | 11.37 | 11.09 | 6.802 | 228 | GTGC | 11.36 | 10.94 | 5.512 |
| 197 | AAAG | 9.19 | 8.95 | 6.722 | 229 | GACC | 9.14 | 8.79 | 5.413 |
| 198 | TGGT | 10.81 | 10.52 | 6.648 | 230 | CGGG | 10.98 | 10.56 | 5.386 |
| 199 | GGCT | 10.91 | 10.62 | 6.614 | 231 | GGCG | 9.24 | 8.88 | 5.375 |
| 200 | ACCG | 10.39 | 10.11 | 6.606 | 232 | AGCA | 8.22 | 7.91 | 5.373 |
| 201 | GACG | 9.06 | 8.81 | 6.547 | 233 | GGGT | 11.08 | 10.65 | 5.292 |
| 202 | CGCT | 12.36 | 12.02 | 6.545 | 234 | GCGC | 10.88 | 10.45 | 5.277 |
| 203 | ACGC | 10.93 | 10.63 | 6.485 | 235 | AGGT | 9.76 | 9.36 | 5.063 |
| 204 | CGGT | 12.28 | 11.94 | 6.452 | 236 | AAGC | 10.41 | 9.98 | 5.024 |
| 205 | GGAA | 8.86 | 8.61 | 6.405 | 237 | GATG | 10.34 | 9.91 | 5.020 |
| 206 | ACCC | 10.22 | 9.92 | 6.355 | 238 | CGAC | 11.57 | 11.08 | 4.886 |
| 207 | CGAA | 11.11 | 10.78 | 6.286 | 239 | CGGC | 11.30 | 10.81 | 4.868 |
| 208 | GCCT | 12.19 | 11.82 | 6.228 | 240 | AGGA | 7.95 | 7.60 | 4.705 |
| 209 | CGAG | 10.88 | 10.55 | 6.198 | 241 | AGCG | 8.32 | 7.95 | 4.689 |
| 210 | CACC | 11.95 | 11.58 | 6.165 | 242 | AGAG | 7.89 | 7.53 | 4.612 |
| 211 | TGGC | 9.26 | 8.97 | 6.134 | 243 | CGCC | 11.28 | 10.75 | 4.505 |
| 212 | AGCT | 9.81 | 9.50 | 6.089 | 244 | GAGG | 9.37 | 8.94 | 4.492 |
| 213 | GAAG | 8.57 | 8.30 | 6.077 | 245 | GAGC | 10.22 | 9.72 | 4.284 |
| 214 | AGAC | 9.22 | 8.92 | 6.057 | 246 | GCCC | 10.14 | 9.64 | 4.096 |
| 215 | CGGA | 10.86 | 10.51 | 6.012 | 247 | GCCG | 10.48 | 9.96 | 4.081 |
| 216 | TGGG | 9.21 | 8.91 | 5.993 | 248 | GGAG | 8.69 | 8.25 | 4.065 |
| 217 | TGAC | 9.64 | 9.33 | 5.972 | 249 | AGGG | 8.45 | 8.00 | 3.694 |
| 218 | AACC | 9.88 | 9.55 | 5.838 | 250 | GGGC | 9.44 | 8.94 | 3.686 |
| 219 | GGTG | 11.17 | 10.79 | 5.802 | 251 | TGCC | 9.01 | 8.53 | 3.641 |
| 220 | AAGG | 9.20 | 8.88 | 5.750 | 252 | GGAC | 9.29 | 8.79 | 3.616 |
| 221 | CGCA | 11.35 | 10.95 | 5.746 | 253 | AGGC | 8.26 | 7.77 | 3.034 |
| 222 | AGAA | 8.33 | 8.04 | 5.691 | 254 | GGCC | 9.25 | 8.69 | 2.997 |
| 223 | GCCA | 10.15 | 9.78 | 5.640 | 255 | AGCC | 9.00 | 8.41 | 2.296 |
| 224 | CCGC | 12.05 | 11.61 | 5.615 | 256 | GGGG | 5.75 | 5.26 | 0.000 |

### << Experiment 3 >>

Among 256 types of 5'-randomized DNAs, 5'-adenylated single-stranded DNAs (SEQ ID NOs: 7 to 10 and 14), whose NNNN in SEQ ID NO: 6 is given by GTAT (No. 3 in Experiment 2), GACA (No. 40 in Experiment 2), AACT (No. 79 in Experiment 2), AACA (No. 118 in Experiment 2), or AGGA (No. 240 in Experiment 2), were prepared.
[SEQ ID NO: 7] 5'-App/GTATGTAGGCACCATCAAT/3'ddC
[SEQ ID NO: 8] 5'-App/GACAGTAGGCACCATCAAT/3'ddC
[SEQ ID NO: 9] 5'-App/AACTGTAGGCACCATCAAT/3'ddC
[SEQ ID NO: 10] 5'-App/AACAGTAGGCACCATCAAT/3'ddC
[SEQ ID NO: 14] 5'-App/AGGAGTAGGCACCATCAAT/3'ddC

Each 5'-Adenylated single-stranded DNA was treated with RecJ as follows.
Nuclease free water 4.3 µL
T4 RNA Ligase Reaction Buffer (NEB/B0216L) 1 µL
100% DMSO (13445-74/Nacalai Tesque) 1 µL
50% PEG (NEB/B1004) 1 µL
500 mM NaCl (31334-51/Nacalai Tesque) 1.2 µL
SUPERase inhibitor (AM2696/Thermo) 0.5 µL
RecJf (M0264S/NEB) 2 µL
1 µM 5'-Adenylated single-stranded DNA 1 µL

The mixes above were incubated at 37°C for 30 minutes, further incubated at 65°C for 20 minutes, and then subjected to acrylamide gel electrophoresis, SYBR Gold staining, and photographing with ImageQuant, to confirm degradation of the individual samples. Note that the prepared acrylamide gel had final concentrations of 20% acrylamide, 1×TBE, and 7M Urea.

### [Results]

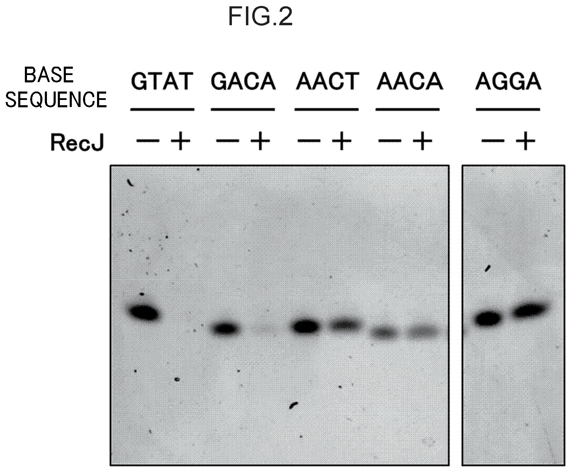

Results are shown in Fig. 2. RecJ was found to be able to degrade the 5'-adenylated single-stranded DNAs, whose NNNNs in SEQ ID NO: 6 are given by GTAT (No. 3 in Experiment 2), GACA (No. 40 in Experiment 2), AACT (No. 79 in Experiment 2), and AACA (No. 118 in Experiment 2), but was unable to degrade the 5'-adenylated single-stranded DNA having AGGA (No. 240 in Experiment 2). Accordingly, degradability by RecJ was found to vary depending on the combination of the 1st to 4th bases in the base sequence at the 5'-end of the 5'-adenylated single-stranded DNA, exclusive of adenylic acid at the 5'-end.

### << Experiment 4 >>

Among 256 types of 5'-randomized DNAs, 5'-adenylated single-stranded DNAs (SEQ ID NOs: 7 to 14), whose NNNN in SEQ ID NO: 6 is given by GTAT (No. 3 in Experiment 2), GACA (No. 40 in Experiment 2), AACT (No. 79 in Experiment 2), AACA (No. 118 in Experiment 2), TCCA (No. 147 in Experiment 2), AACG (No. 167 in Experiment 2), CCCA (No. 196 in Experiment 2), or AGGA (No. 240 in Experiment 2), were prepared.
[SEQ ID NO: 7] 5'-App/GTATGTAGGCACCATCAAT/3'ddC
[SEQ ID NO: 8] 5'-App/GACAGTAGGCACCATCAAT/3'ddC
[SEQ ID NO: 9] 5'-App/AACTGTAGGCACCATCAAT/3'ddC
[SEQ ID NO: 10] 5'-App/AACAGTAGGCACCATCAAT/3'ddC
[SEQ ID NO: 11]5'-App/TCCAGTAGGCACCATCAAT/3'ddC
[SEQ ID NO: 12]5'-App/AACGGTAGGCACCATCAAT/3'ddC
[SEQ ID NO: 13]5'-App/CCCAGTAGGCACCATCAAT/3'ddC
[SEQ ID NO: 14] 5'-App/AGGAGTAGGCACCATCAAT/3'ddC

With use of the 5'-adenylated single-stranded DNA as the 3' adapter, the NGS library was prepared by subjecting the total RNA to be contained in the library. Note as described previously, method for preparing the NGS library was conducted referring to Seda Eminaga et al., Quantification of microRNA Expression with Next-Generation Sequencing, Curr Protoc Mol Biol. 2013 July; 04: Unit 4.17., unless otherwise specified. More specifically, [1] Ligation of 3'Adapter, [2] Degradation of Excessive 3'Adapter, [3] Ligation of 5'Adapter, [4] Reverse Transcription, and [5] PCR were conducted in this order, in the same manner as in << Experiment 1 >>.

The solution obtained by the operations up to [5] above was purified with use of AMPure XP (Beckman Coulter/A63881), to complete the preparation of the NGS library. The completed library was sequenced on Illumina Nextseq550, and data was analyzed by QIAGEN GeneGlobe, to map known microRNA sequences.

### [Results]

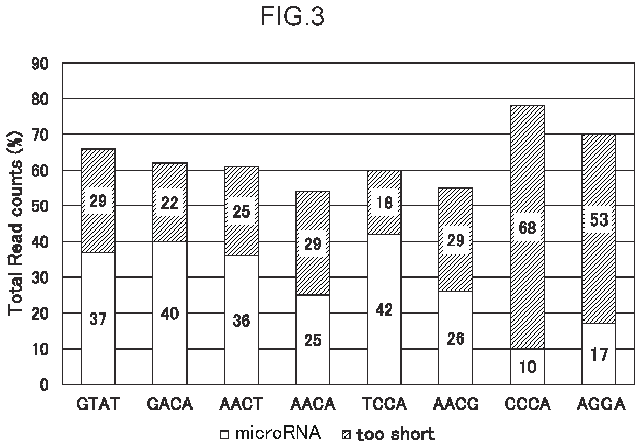

Fig. 3 shows the rate (%) of microRNA read counts, and the rate (%) of too short read counts, relative to the total read counts. The too short read counts herein mean the read counts of adapter dimer formed of the 3' adapter and the 5' adapter.

Accordingly, in a case where the 5'-adenylated single-stranded DNA, whose NNNN in SEQ ID NO: 6 is given by GTAT (No. 3 in Experiment 2), GACA (No. 40 in Experiment 2), AACT (No. 79 in Experiment 2), AACA (No. 118 in Experiment 2), TCCA (No. 147 in Experiment 2), or AACG (No. 167 in Experiment 2) is used as the 3' adapter, the rate of too short read counts was found to be 50% or less, proving that preparation of the NGS library was feasible.

From the above, it was confirmed that RecJ degraded the 5'-adenylated single-stranded DNA, in a case where the 5'-adenylated single-stranded DNA has specific base sequences, in the base sequence at the 5'-end exclusive of adenylic acid at the 5'-end, and also made it possible to prepare the NGS library.

The disclosure of Japanese Patent Application No. 2023-177841 filed on October 13, 2023 is incorporated herein by reference in its entirety. All documents, patent applications, and technical standards described in this specification are incorporated herein by reference to the same extent as if each document, patent application, and technical standard were specifically and individually indicated to be incorporated by reference.

## Claims

1. A method for degrading 5'-adenylated single-stranded DNA, the method comprising degrading 5'-adenylated single-stranded DNA with RecJ, with a base sequence at a 5'-end of the 5'-adenylated single-stranded DNA, exclusive of adenylic acid at the 5'-end, being any one of the base sequences listed in the following Table A:
**Table A**
| No. | Base sequence (5' to 3') | No. | Base sequence (5' to 3') | No. | Base sequence (5' to 3') | No. | Base sequence (5' to 3') |
|---|---|---|---|---|---|---|---|
| 1 | GTTA | 46 | TTTC | 91 | TATG | 136 | CCTA |
| 2 | TAAT | 47 | ACAT | 92 | GAGA | 137 | GAGT |
| 3 | GTAT | 48 | GCTA | 93 | TGAT | 138 | AGTA |
| 4 | TGTT | 49 | TCTG | 94 | CTCA | 139 | CGAT |
| 5 | GTTT | 50 | ATTG | 95 | CATA | 140 | CCGT |
| 6 | TTAA | 51 | ATAA | 96 | CTAA | 141 | GGCA |
| 7 | GTTC | 52 | CGTT | 97 | GCAA | 142 | CATG |
| 8 | TACT | 53 | CTTC | 98 | TTGG | 143 | AAGT |
| 9 | CTTT | 54 | GTTG | 99 | CTAG | 144 | ACAA |
| 10 | TCAT | 55 | GTCG | 100 | TAAC | 145 | AGTC |
| 11 | TATA | 56 | CTTG | 101 | TAAA | 146 | CGTA |
| 12 | GATT | 57 | TGTA | 102 | ATCC | 147 | TCCA |
| 13 | TATC | 58 | ACTA | 103 | TCAG | 148 | CCCT |
| 14 | TTTG | 59 | GAAT | 104 | ACTG | 149 | TACC |
| 15 | TTCA | 60 | TCCT | 105 | ATAG | 150 | GGTC |
| 16 | TTAC | 61 | TTTT | 106 | CCTT | 151 | ACCA |
| 17 | TTCT | 62 | GACT | 107 | GAAA | 152 | CGTC |
| 18 | CATT | 63 | TAGT | 108 | CTGA | 153 | TAGG |
| 19 | TCTA | 64 | CAAT | 109 | CCAT | 154 | AAAA |
| 20 | TTGT | 65 | ATTC | 110 | GCGT | 155 | ACAG |
| 21 | TTCC | 66 | TCTC | 111 | TCAA | 156 | CACA |
| 22 | GTCA | 67 | AAAT | 112 | CATC | 157 | ACAC |
| 23 | CTAT | 68 | CTCT | 113 | TTGC | 158 | CGTG |
| 24 | AGTT | 69 | GTAG | 114 | CTAC | 159 | ATGC |
| 25 | ATTT | 70 | ATGT | 115 | ACGT | 160 | TCCG |
| 26 | TTAT | 71 | ATAC | 116 | TCGG | 161 | GCAG |
| 27 | GTCT | 72 | AATA | 117 | ACTC | 162 | ATGG |
| 28 | GTAA | 73 | ATGA | 118 | AACA | 163 | CAGA |
| 29 | ATAT | 74 | TTGA | 119 | TGTG | 164 | TAGA |
| 30 | GCTT | 75 | TCAC | 120 | CTGG | 165 | TAGC |
| 31 | CTTA | 76 | TCGT | 121 | TCGC | 166 | TGCT |
| 32 | GGTT | 77 | ACTT | 122 | ACGA | 167 | AACG |
| 33 | TCTT | 78 | GGTA | 123 | CAGT | 168 | CAAA |
| 34 | TTAG | 79 | AACT | 124 | CTCC | 169 | TGAG |
| 35 | ATCT | 80 | TACA | 125 | TCCC | 170 | ACGG |
| 36 | GATA | 81 | CTCG | 126 | TCGA | 171 | TGAA |
| 37 | TATT | 82 | GCTC | 127 | CTGC | 172 | AAGA |
| 38 | ATTA | 83 | AATC | 128 | GCTG | 173 | CACG |
| 39 | GTGA | 84 | GCAT | 129 | TGTC | 174 | GGAT |
| 40 | GACA | 85 | CACT | 130 | CCTG | 175 | TGGA |
| 41 | TTTA | 86 | GTAC | 131 | TACG | 176 | AGTG |
| 42 | AATT | 87 | GCGA | 132 | GTGG | 177 | CCAC |
| 43 | TTCG | 88 | ATCG | 133 | CCTC | 178 | GTCC |
| 44 | GATC | 89 | GTGT | 134 | ACCT | 179 | AATG |
| 45 | ATCA | 90 | CTGT | 135 | TAAG | 180 | GCGG |

2. The method for degrading 5'-adenylated single-stranded DNA according to claim 1, wherein the base sequence at the 5'-end is any one selected from the group consisting of the base sequence Nos. 1 to 118 listed in the Table A.

3. The method for degrading 5'-adenylated single-stranded DNA according to claim 1 or 2, wherein, in a case in which any enzyme other than RecJ is also present, the enzyme other than RecJ allows the 5'-adenylated single-stranded DNA to remain adenylated, during the degrading.

4. A method for ligating a 3' adapter to a nucleic acid, the method comprising:
ligating 5'-adenylated single-stranded DNA, as the 3' adapter, to a 3'-end of the nucleic acid; and
after the ligating of the 5'-adenylated single-stranded DNA, degrading excessive 5'-adenylated single-stranded DNA by the method for degrading according to claim 1 or 2.

5. A method for preparing an NGS library, the method comprising:
ligating 5'-adenylated single-stranded DNA, as a 3' adapter, to each 3'-end of a plurality of types of RNA having different sequences;
after the ligating of the 5'-adenylated single-stranded DNA, degrading excessive 5'-adenylated single-stranded DNA by the method for degrading according to claim 1 or 2;
ligating single-stranded RNA, as a 5' adapter, to each 5'-end of the plurality of types of RNA;
synthesizing cDNA from the plurality of types of RNA ligated with the 3' adapter and the 5' adapter by a reverse transcription reaction; and
amplifying the obtained cDNA by PCR.

6. The method for preparing an NGS library according to claim 5, wherein, in the degrading, in a case in which any enzyme other than RecJ is also present, the enzyme other than RecJ allows the 5'-adenylated single-stranded DNA to remain adenylated.

7. The method for preparing an NGS library according to claim 5, wherein the plurality of types of RNA are small RNAs.

8. A kit for preparing an NGS library, the kit comprising: 5'-adenylated single-stranded DNA stored in a first container; and RecJ stored in a second container, with a base sequence at a 5'-end of the 5'-adenylated single-stranded DNA, exclusive of adenylic acid at the 5'-end, being any one of the base sequences listed in the following Table A:
**Table A**
| No. | Base sequence (5' to 3') | No. | Base sequence (5' to 3') | No. | Base sequence (5' to 3') | No. | Base sequence (5' to 3') |
|---|---|---|---|---|---|---|---|
| 1 | GTTA | 46 | TTTC | 91 | TATG | 136 | CCTA |
| 2 | TAAT | 47 | ACAT | 92 | GAGA | 137 | GAGT |
| 3 | GTAT | 48 | GCTA | 93 | TGAT | 138 | AGTA |
| 4 | TGTT | 49 | TCTG | 94 | CTCA | 139 | CGAT |
| 5 | GTTT | 50 | ATTG | 95 | CATA | 140 | CCGT |
| 6 | TTAA | 51 | ATAA | 96 | CTAA | 141 | GGCA |
| 7 | GTTC | 52 | CGTT | 97 | GCAA | 142 | CATG |
| 8 | TACT | 53 | CTTC | 98 | TTGG | 143 | AAGT |
| 9 | CTTT | 54 | GTTG | 99 | CTAG | 144 | ACAA |
| 10 | TCAT | 55 | GTCG | 100 | TAAC | 145 | AGTC |
| 11 | TATA | 56 | CTTG | 101 | TAAA | 146 | CGTA |
| 12 | GATT | 57 | TGTA | 102 | ATCC | 147 | TCCA |
| 13 | TATC | 58 | ACTA | 103 | TCAG | 148 | CCCT |
| 14 | TTTG | 59 | GAAT | 104 | ACTG | 149 | TACC |
| 15 | TTCA | 60 | TCCT | 105 | ATAG | 150 | GGTC |
| 16 | TTAC | 61 | TTTT | 106 | CCTT | 151 | ACCA |
| 17 | TTCT | 62 | GACT | 107 | GAAA | 152 | CGTC |
| 18 | CATT | 63 | TAGT | 108 | CTGA | 153 | TAGG |
| 19 | TCTA | 64 | CAAT | 109 | CCAT | 154 | AAAA |
| 20 | TTGT | 65 | ATTC | 110 | GCGT | 155 | ACAG |
| 21 | TTCC | 66 | TCTC | 111 | TCAA | 156 | CACA |
| 22 | GTCA | 67 | AAAT | 112 | CATC | 157 | ACAC |
| 23 | CTAT | 68 | CTCT | 113 | TTGC | 158 | CGTG |
| 24 | AGTT | 69 | GTAG | 114 | CTAC | 159 | ATGC |
| 25 | ATTT | 70 | ATGT | 115 | ACGT | 160 | TCCG |
| 26 | TTAT | 71 | ATAC | 116 | TCGG | 161 | GCAG |
| 27 | GTCT | 72 | AATA | 117 | ACTC | 162 | ATGG |
| 28 | GTAA | 73 | ATGA | 118 | AACA | 163 | CAGA |
| 29 | ATAT | 74 | TTGA | 119 | TGTG | 164 | TAGA |
| 30 | GCTT | 75 | TCAC | 120 | CTGG | 165 | TAGC |
| 31 | CTTA | 76 | TCGT | 121 | TCGC | 166 | TGCT |
| 32 | GGTT | 77 | ACTT | 122 | ACGA | 167 | AACG |
| 33 | TCTT | 78 | GGTA | 123 | CAGT | 168 | CAAA |
| 34 | TTAG | 79 | AACT | 124 | CTCC | 169 | TGAG |
| 35 | ATCT | 80 | TACA | 125 | TCCC | 170 | ACGG |
| 36 | GATA | 81 | CTCG | 126 | TCGA | 171 | TGAA |
| 37 | TATT | 82 | GCTC | 127 | CTGC | 172 | AAGA |
| 38 | ATTA | 83 | AATC | 128 | GCTG | 173 | CACG |
| 39 | GTGA | 84 | GCAT | 129 | TGTC | 174 | GGAT |
| 40 | GACA | 85 | CACT | 130 | CCTG | 175 | TGGA |
| 41 | TTTA | 86 | GTAC | 131 | TACG | 176 | AGTG |
| 42 | AATT | 87 | GCGA | 132 | GTGG | 177 | CCAC |
| 43 | TTCG | 88 | ATCG | 133 | CCTC | 178 | GTCC |
| 44 | GATC | 89 | GTGT | 134 | ACCT | 179 | AATG |
| 45 | ATCA | 90 | CTGT | 135 | TAAG | 180 | GCGG |

9. The kit for preparing an NGS library according to claim 8, wherein a nucleotide at a 3'-end of the 5'-adenylated single-stranded DNA is modified with a compound having a modification structure capable of suppressing binding of nucleic acid.

10. The kit for preparing an NGS library according to claim 8 or 9, further comprising at least one selected from the group consisting of 5' adapter, ligase, reverse transcriptase, reverse transcription primer, PCR enzyme, and PCR primer, in a manner such that each is contained in a separate container.

11. A 3' adapter for preparing a nucleic acid library, the 3' adapter being a single-stranded DNA having a nucleotide at a 5'-end that is adenylated, and a base sequence at the 5'-end of the single-stranded DNA, exclusive of adenylic acid at the 5'-end, being any one of the base sequences listed in the following Table A:
**Table A**
| No. | Base sequence (5' to 3') | No. | Base sequence (5' to 3') | No. | Base sequence (5' to 3') | No. | Base sequence (5' to 3') |
|---|---|---|---|---|---|---|---|
| 1 | GTTA | 46 | TTTC | 91 | TATG | 136 | CCTA |
| 2 | TAAT | 47 | ACAT | 92 | GAGA | 137 | GAGT |
| 3 | GTAT | 48 | GCTA | 93 | TGAT | 138 | AGTA |
| 4 | TGTT | 49 | TCTG | 94 | CTCA | 139 | CGAT |
| 5 | GTTT | 50 | ATTG | 95 | CATA | 140 | CCGT |
| 6 | TTAA | 51 | ATAA | 96 | CTAA | 141 | GGCA |
| 7 | GTTC | 52 | CGTT | 97 | GCAA | 142 | CATG |
| 8 | TACT | 53 | CTTC | 98 | TTGG | 143 | AAGT |
| 9 | CTTT | 54 | GTTG | 99 | CTAG | 144 | ACAA |
| 10 | TCAT | 55 | GTCG | 100 | TAAC | 145 | AGTC |
| 11 | TATA | 56 | CTTG | 101 | TAAA | 146 | CGTA |
| 12 | GATT | 57 | TGTA | 102 | ATCC | 147 | TCCA |
| 13 | TATC | 58 | ACTA | 103 | TCAG | 148 | CCCT |
| 14 | TTTG | 59 | GAAT | 104 | ACTG | 149 | TACC |
| 15 | TTCA | 60 | TCCT | 105 | ATAG | 150 | GGTC |
| 16 | TTAC | 61 | TTTT | 106 | CCTT | 151 | ACCA |
| 17 | TTCT | 62 | GACT | 107 | GAAA | 152 | CGTC |
| 18 | CATT | 63 | TAGT | 108 | CTGA | 153 | TAGG |
| 19 | TCTA | 64 | CAAT | 109 | CCAT | 154 | AAAA |
| 20 | TTGT | 65 | ATTC | 110 | GCGT | 155 | ACAG |
| 21 | TTCC | 66 | TCTC | 111 | TCAA | 156 | CACA |
| 22 | GTCA | 67 | AAAT | 112 | CATC | 157 | ACAC |
| 23 | CTAT | 68 | CTCT | 113 | TTGC | 158 | CGTG |
| 24 | AGTT | 69 | GTAG | 114 | CTAC | 159 | ATGC |
| 25 | ATTT | 70 | ATGT | 115 | ACGT | 160 | TCCG |
| 26 | TTAT | 71 | ATAC | 116 | TCGG | 161 | GCAG |
| 27 | GTCT | 72 | AATA | 117 | ACTC | 162 | ATGG |
| 28 | GTAA | 73 | ATGA | 118 | AACA | 163 | CAGA |
| 29 | ATAT | 74 | TTGA | 119 | TGTG | 164 | TAGA |
| 30 | GCTT | 75 | TCAC | 120 | CTGG | 165 | TAGC |
| 31 | CTTA | 76 | TCGT | 121 | TCGC | 166 | TGCT |
| 32 | GGTT | 77 | ACTT | 122 | ACGA | 167 | AACG |
| 33 | TCTT | 78 | GGTA | 123 | CAGT | 168 | CAAA |
| 34 | TTAG | 79 | AACT | 124 | CTCC | 169 | TGAG |
| 35 | ATCT | 80 | TACA | 125 | TCCC | 170 | ACGG |
| 36 | GATA | 81 | CTCG | 126 | TCGA | 171 | TGAA |
| 37 | TATT | 82 | GCTC | 127 | CTGC | 172 | AAGA |
| 38 | ATTA | 83 | AATC | 128 | GCTG | 173 | CACG |
| 39 | GTGA | 84 | GCAT | 129 | TGTC | 174 | GGAT |
| 40 | GACA | 85 | CACT | 130 | CCTG | 175 | TGGA |
| 41 | TTTA | 86 | GTAC | 131 | TACG | 176 | AGTG |
| 42 | AATT | 87 | GCGA | 132 | GTGG | 177 | CCAC |
| 43 | TTCG | 88 | ATCG | 133 | CCTC | 178 | GTCC |
| 44 | GATC | 89 | GTGT | 134 | ACCT | 179 | AATG |
| 45 | ATCA | 90 | CTGT | 135 | TAAG | 180 | GCGG |

12. The 3' adapter for preparing a nucleic acid library according to claim 11, wherein the base sequence at the 5'-end is any one selected from the group consisting of the base sequence Nos. 1 to 118 listed in the Table A.

13. The 3' adapter for preparing a nucleic acid library according to claim 11 or 12, wherein a nucleotide at a 3'-end of the single-stranded DNA is modified with a compound having a modification structure capable of suppressing binding of nucleic acid.
